# FASCICULE DE BREVET EUROPEEN

(11) **EP 3 727 144 B1**
(45) Date de publication et mention de la délivrance du brevet: **08.04.2026**
(21) Numéro de dépôt: 18833663.0
(22) Date de dépôt: 21.12.2018
(51) Int. Cl.: A61B 5/021, A61B 5/00, A61B 5/024, A61B 5/1455, A61B 5/022

(54) **MÉTHODE DE MESURE DE LA PRESSION ARTÉRIELLE MOYENNE**
SYSTEM ZUR MESSUNG DES MITTLEREN ARTERIENDRUCKS
SYSTEM FOR MEASURING THE MEAN ARTERIAL PRESSURE

(30) Priorité: 22.12.2017 FR 1762978; 29.12.2017 FR 1763405
(43) Date de publication de la demande: 28.10.2020
(73) Titulaire: Assistance Publique Hôpitaux de Paris, 75012 Paris (FR)
(72) Inventeur: VALLÉE, Fabrice, 75010 PARIS (FR); JOACHIM, Jona, 75010 PARIS (FR); COUTROT, Maxime, 75010 PARIS (FR); MATEO, Joaquim, 75010 PARIS (FR); GAYAT, Étienne, 75010 PARIS (FR); MEBAZAA, Alexandre, 75010 PARIS (FR)
(74) Mandataire: Flesselles, Bruno F.G.
(86) Numéro de dépôt international: PCT/EP2018/086748
(87) Numéro de publication internationale: WO 2019/122406

(56) Documents cités:
- WO-A1-94/17728
- US-A1- 2007 055 163
- US-A1- 2011 009 754
- ERIC S WINOKUR ET AL: "A wearable vital signs monitor at the ear for continuous heart rate and Pulse Transit Time measurements", ENGINEERING IN MEDICINE AND BIOLOGY SOCIETY (EMBC), 2013 35TH ANNUAL INTERNATIONAL CONFERENCE OF THE IEEE, IEEE, 28 August 2012 (2012-08-28), pages 2724 - 2727, XP032463504, ISSN: 1557-170X, DOI: 10.1109/EMBC.2012.6346527
- COHEN ZACHARY ET AL: "Optical-Based Sensor Prototype for Continuous Monitoring of the Blood Pressure", IEEE SENSORS JOURNAL, IEEE SERVICE CENTER, NEW YORK, NY, US, vol. 17, no. 13, 1 July 2017 (2017-07-01), pages 4258 - 4268, XP011652521, ISSN: 1530-437X, [retrieved on 20170610], DOI: 10.1109/JSEN.2017.2704098

## Description

### DOMAINE TECHNIQUE

La présente invention se rapporte au domaine de l'anesthésie, et plus particulièrement à une nouvelle procédure et un nouvel algorithme utilisable notamment pour suivre l'état général d'un patient durant une anesthésie et en particulier pendant la période de l'induction de l'anesthésie.

### ETAT DE LA TECHNIQUE ANTERIEUR

Le rôle de l'anesthésiste-réanimateur est de veiller au et d'assurer le contrôle des grandes fonctions vitales du patient, lors d'une anesthésie générale, pendant toute la durée de cette anesthésie, et surtout la période de l'induction, période critique où l'emploi de produit anesthésiant à forte dose afin d'induire la perte de conscience entraine le plus souvent une hypotension artérielle.

Cette hypotension artérielle résulte de l'interaction entre l'effet périphérique direct vasodilatateur des drogues hypnotiques et morphinomimétiques, et l'effet central anesthésique des centres de régulation de la pression artérielle. En effet, la pression artérielle (PA) est un paramètre physiologique finement régulé et compensé par des systèmes reflexes complexes. Au cours de l'anesthésie générale tous ces systèmes sont globalement inhibés et ce de manière proportionnelle à la profondeur de l'anesthésie. Le risque lié à l'hypotension artérielle est démontré, mais le seuil à partir duquel apparaissent des conséquences pour la perfusion d'un ou plusieurs organes varie en fonction du mécanisme, des anomalies associées (FC, débit cardiaque et transport en oxygène) et du terrain particulier du patient.

Le rôle majeur de la pression artérielle moyenne dans la perfusion des organes, notamment celle des organes les plus sensibles à l'hypoperfusion (cerveau, cœur, tube digestif et reins) expose à un risque de défaillance en cas de baisse de celle-ci.

En pratique clinique, la prévention de l'hypotension artérielle peropératoire permettant de maintenir une pression artérielle moyenne proche de la pression artérielle moyenne initiale, c'est-à-dire avant l'anesthésie, est largement pratiquée afin de diminuer le risque vital et l'amélioration des suites opératoires. De plus, on utilise des agents vasoactifs au cours de l'anesthésie générale afin de corriger au plus vite toute variation de pression artérielle moyenne potentiellement délétère, en particulier la phényléphrine, l'éphédrine et la noradrénaline.

On peut couramment mesurer trois pressions artérielles : Les pressions artérielles systolique (PAS) et diastolique (PAD) sont mesurées à l'aide d'un brassard placé au niveau du bras et d'un stéthoscope, le médecin écoutant l'apparition puis la disparition du pouls. Ces pressions représentent respectivement les pressions maximale systolique et minimale diastolique au cours d'un cycle cardiaque. La pression artérielle moyenne (PAM) est mesurée à l'aide d'un sphygmomanomètre et est indicatrice de la pression de perfusion des organes.

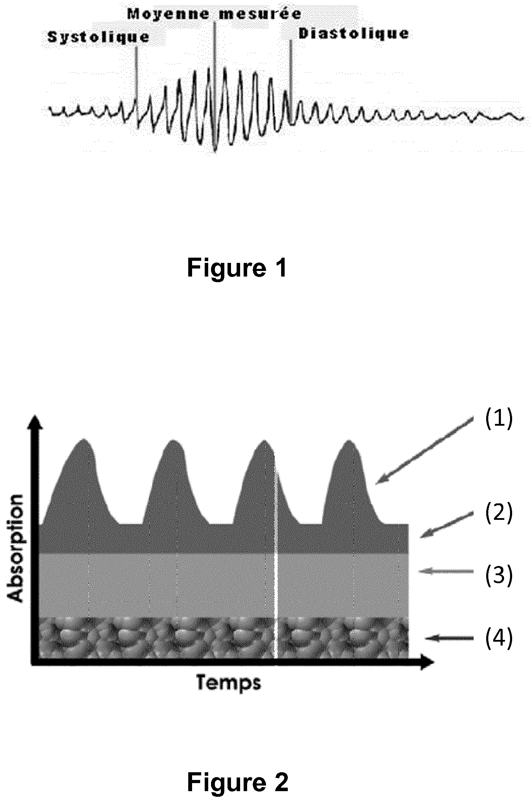

Le sphygmomanomètre (ou électrocardioscope tensiomètre électronique) n'utilise pas le principe de la méthode auscultatoire ou palpatoire, mais une mesure oscillométrique. Lorsque le manchon placé au niveau de l'avant-bras du patient se dégonfle automatiquement, des oscillations sont enregistrées par l'appareil. Ces oscillations débutent avant la valeur réelle de la systolique et prennent fin après la valeur réelle de la diastolique. La valeur maximale de l'oscillation représente la pression artérielle moyenne (figure 1). À partir de cette valeur de PAM et d'algorithmes développés par les fabricants, les PAS et PAD sont calculées. C'est cet appareil qui est utilisé par les anesthésistes dans les blocs opératoires.

On peut estimer la pression artérielle moyenne à partir des pressions artérielles systolique et diastolique par la formule PAM = 2/3 PAD + 1/3 PAS. Toutefois cette formule n'est qu'une estimation, discutée dans l'art, d'autres formules ayant notamment été développées (voir en particulier Razminia et al Catheter Cardiovasc Interv. 2004 Dec;63(4):419-25). Dans une réponse donnée sur Research Gate, Gianni Losano (Université Turin) rappelle la définition de la pression artérielle moyenne (moyenne de toutes les valeurs que la tension artérielle présente pendant tout un cycle cardiaque), et qu'elle peut parfois être la moyenne entre la pression systolique et la pression diastolique.
https://www.researchgate.net/post/What_formulas_and_methods_exist_for_the_ca lculation_of_mean_arterial_blood_pressure
il apparaît donc que la connaissance des pressions systolique et diastolique est insuffisante pour connaître la pression artérielle moyenne.

L'objectif de l'anesthésiologiste est donc de protéger le patient des perturbations physiologiques induites par l'acte chirurgical et les agents anesthésiques. Elle nécessite une surveillance continue du système cardiovasculaire (fréquence cardiaque, pression artérielle), du système respiratoire (fréquence respiratoire, oxymètre de pouls, CO2 expiré) et de la température en cas d'intervention de longue durée. Ce contrôle doit être adapté aux risques et aux contextes pour limiter ces épisodes d'instabilité hémodynamique.

En routine, le monitorage minimum comprend notamment la mesure au minimum toutes les 5 minutes de la pression artérielle moyenne, de manière invasive ou non, ainsi que la mesure continue de la saturation pulsée en oxygène (SpO2). Ces contrôles sont imposés par la réglementation.

La mesure de la pression artérielle moyenne non invasive est aujourd'hui réalisée par méthode oscillométrique à l'aide d'un brassard, ainsi qu'indiqué plus haut. Cependant, l'une des limites majeures de ce contrôle de pression artérielle est son caractère intermittent et donc systématiquement en retard par rapport aux variations hémodynamiques engendrées du fait de l'anesthésie. Ainsi, il n'est pas possible d'obtenir les valeurs de la pression artérielle en continu. En effet, la mesure de la pression artérielle prend environ une minute et ne peut être répétée trop souvent, pour ne pas léser le bras du patient par des gonflages trop répétés.

La mesure de la saturation pulsée en oxygène est effectuée par pléthysmographie avec un capteur généralement positionné au bout du doigt. Cette mesure est peu couteuse et permet d'obtenir des informations en continu.

Ce capteur est appelé oxymètre de pouls (pléthysmographe), et contient deux diodes émettant une lumière rouge qui doit être située face à une zone réceptrice. Les diodes émettent deux lumières (rouge et infrarouge), dont on mesure leur absorption par le flux pulsatile. Cette absorption des lumières rouge et infrarouge est variable selon qu'elle rencontre l'hémoglobine réduite (Hb) non oxygénée ou de l'oxyhémoglobine oxygénée (HbO2). Les données de sortie sont donc des données d'absorption. La figure 2 montre que l'absorption mesurée par l'oxymètre de pouls contient plusieurs composantes, la seule variable observée dans des conditions stables étant le flux systolique artériel.

L'onde de pouls est générée par le cœur à chaque battement. Elle entraîne des variations de volume sanguin dans les artères, qui se contractent et se détendent lors de son passage. Cette onde est dicrote, reflétant (premier pic) l'expulsion sanguine du ventricule dans le système cardiovasculaire (pression systolique), et (second pic, ou changement de la pente de décroissance de l'onde de pouls après le maximum) la propulsion résiduelle observée à la fermeture des valves sigmoïdes, et représentant la relaxation (contraction) des artères proches du coeur qui s'étaient dilatées pour absorber l'afflux sanguin lors de la propulsion sanguine.
Il est également rappelé que l'onde de pression artérielle peut être décrite selon sa composante moyenne et sa composante pulsatile. La composante moyenne est la PAM, qui est considérée constante de l'aorte aux artères périphériques de gros calibre alors que la composante pulsatile varie selon des phénomènes complexes tout le long de l'arbre artériel.

Une application pour téléphones intelligents existe déjà en tant qu'aide pour le suivi de certains paramètres physiologiques (Captesia, décrite notamment par Desebbe et al, Anesth Analg 2016;123:105-13). Cette application demande la prise d'une photographie du graphe de l'onde de pouls, tel qu'apparaissant par pléthysmographie, et l'envoi de cette photo à un serveur, avec certains paramètres du patient. L'application renvoie la variation de la pression du pouls, mais pas la pression artérielle moyenne en temps réel et continu, car demandant une intervention de l'opérateur. Cette application est liée à la demande de brevet WO 2015/181622.

La demande WO 2017/037369 décrit un dispositif non invasif de mesure du débit aortique chez un petit mammifère, utilisant un dispositif de pléthysmographie du thorax par inductancemétrie, l'acquisition et l'analyse du signal de variation de section de chaque spire.

La demande FR3024943 décrit un procédé de détermination de la fréquence respiratoire d'un individu à partir de son rythme cardiaque, par mesure du signal cardiaque par pléthysmographie.

La demande WO 2007/128518 décrit un dispositif non-invasif de mesure en continu de la pression artérielle (PA) caractérisé en ce qu'il comprend des moyens de mesure indirecte à partir d'un signal (VS) représentant directement ou indirectement les variations de volume de sang dans un organe ou une partie du corps, ledit signal de variation de volume étant calibré à l'aide des valeurs intermittentes de PA obtenues par des méthodes non-invasives standard, de préférence sans utiliser une ou plusieurs constantes prédéfinies, et en particulier un ou plusieurs paramètres physiologiques supposés constants. Le signal (VS) représentant directement ou indirectement les variations de volume de sang dans un organe ou une partie du corps peut être l'onde pléthysmographique de la saturation en oxygène du sang (SpO2) mesurée à l'aide d'un saturomètre au niveau d'un doigt de la main ou du pied, de l'oreille, du front, des ailes du nez, ou d'autre organe ou partie du corps. Ce dispositif peut comprendre des moyens pour détecter l'amplitude du signal VS et le temps pour lequel le signal VS atteint son maximum (VSmax) et/ou son minimum (VSmin) et/ou une valeur de référence prédéfinie (VSO), lors de chaque battement (cycle) cardiaque, en calculant en temps réel les paramètres suivants :
- le temps de montée (Tm) pour chaque battement (cycle) cardiaque, défini comme l'intervalle de temps du passage du signal VS de la valeur VSmin ou la valeur VSO à la valeur Vsmax lors de sa montée: Tm = t( VSmax) - t( VSmin) ou Tm = t(VSmax) - t(VSO) ;
- et/ou le temps de descente (Td) pour chaque battement (cycle) cardiaque, défini comme l'intervalle de temps du passage du signal VS de la valeur VSmax ou la valeur VSO à la valeur VSmin lors de sa descente: Tm = t(VSmin) - t(VSmax) ou Tm = t(VSmin) - t(VSO).

Ce document ne décrit toutefois que l'évaluation de la pression artérielle systolique en utilisant l'amplitude de signal et la durée de la montée de pic avec une calibration par brassard. Toutefois, ainsi que mentionné, cette calibration n'est pas correcte, car le brassard ne donne qu'une estimation de la pression systolique. Par ailleurs, ce document est silencieux sur la façon d'obtenir la fonction liant les paramètres mesurés sur l'onde de pouls et la pression systolique. Il apparaît donc que l'enseignement de ce document n'est pas pertinent pour résoudre le problème adressé dans la présente demande (mesure de la pression artérielle moyenne, déterminante pour la perfusion d'organe) car la pression systolique périphérique n'est pas un bon indicateur de la perfusion tissulaire. De plus, la pression systolique dépend très largement de l'endroit où elle est mesurée. On peut ainsi voir, sur la figure 1, que la systole sur la pléthysmographie ne varie pas de la même façon que la systole réelle. On note par ailleurs que l'équation proposée par ce document utilise une constance, pour prendre en compte la pression diastolique (le calcul est effectué sur la pression pulsée) et qui représente plus ou moins le volume de sang qui a été pompé et rejeté par le coeur. L'utilisation de cette constante ajoute à l'incertitude de la méthode de ce document.

Le brevet US 5269310 décrit un procédé de détermination de la pression artérielle, en attachant un pléthysmographe à un patient de telle sorte que ledit pléthysmographe interagit avec une artère dudit patient, ledit pléthysmographe générant un signal de sortie ayant une relation prédéterminée avec une caractéristique du sang dans ladite artère; étalonner ledit pléthysmographe au cours d'une période d'étalonnage en déterminant la tension artérielle réelle du patient au moyen d'autres moyens que ledit pléthysmographe, puis déterminer la valeur d'une première caractéristique artérielle dans une relation prédéterminée entre ladite première caractéristique artérielle, le volume artériel indiqué par ledit signal de sortie du pléthysmographe, une valeur de conversion correspondant au volume artériel à pression infinie, et ladite tension artérielle réelle au cours de ladite période d'étalonnage; et analyser ledit signal de sortie du pléthysmographe pendant une période de mesure pour déterminer une tension artérielle correspondant audit signal de sortie conformément à ladite relation prédéterminée. Ce document enseigne la mesure de tensions électriques correspondant aux systole et diastole (colonne 6) et à un calcul d'une tension électrique correspondant à la « tension électrique moyenne ». Les méthodes décrites ne mentionnent pas l'obtention ni l'utilisation de la pression artérielle moyenne.

Le brevet US 5309916 décrit un procédé d'évaluation de la pression artérielle, sur la base de deux variables, et décrit essentiellement l'utilisation de la vitesse de l'onde de pouls et de la vitesse du flux sanguin.

La demande US 20070055163 décrit l'utilisation d'un signal de pléthysmographie pour la mesure de la pression artérielle. Les auteurs appliquent une pression externe au patient et mesurent le signal obtenu, ce qui permet de calibrer le système. Toutefois, ce document ne mentionne pas que l'on peut utiliser la mesure de l'onde dicrote pour mesurer ou estimer la pression artérielle moyenne.

La demande US 20110009754 se rapporte au calcul de la pression artérielle, et mentionne la mesure de divers paramètres. Ce document mentionne en particulier le fait de mesurer le maximum de l'onde de pouls ou la durée entre le début de l'onde de pouls et l'onde dicrote (figure 7.E). Ainsi, ce document est plus intéressé par les temps d'arrivée des signaux de pléthysmographie. Ceci est explicite au paragraphe [0144], qui indique que « *The velocity of the pressure pulsation traversing the arteries is positively correlated with systolic blood pressure. Therefore,* as *explained above, measures of pulse arrival time (PAT), and metrics indicative of PAT, can be used to estimate arterial blood pressure ».* Ainsi, ce document n'envisage pas de mesurer la hauteur de l'onde dicrote, ni ne suggère que cet élément pourrait être intéressant, malgré le nombre important de paramètres suggérés.

Winokur et al (Conf Proc IEEE Eng Med Biol Soc. 2012;2012:2724-7) se rapporte à un appareil surveillant l'électrocardiogramme (ECG), le ballistocardiogramme (BCG) et les photopléthysmogrammes (PPG) avec deux sources de lumière. Ce document indique que le temps de transit des impulsions (Pulse Transit Time, PTT) extrait de la corrélation croisée entre le PPG et le BCG montre des résultats améliorés par rapport à la méthode du temps d'arrivée des impulsions (pulse arrival time, PAT, envisagée dans US 20110009754) pour suivre les changements dans la pression artérielle moyenne.

Zachary Cohen (IEEE Sensors Journal, Volume: 17, Issue: 13, July1, 1 2017) décrit un prototype de capteur annulaire pour la mesure continue de la pression sanguine. Ce document décrit une corrélation linéaire entre la tension mesurée en volt de chaque battement cardiaque et la pression sanguine. Ce document décrit les mesures de pressions systolique et diastolique.

### EXPOSE DE L'INVENTION

L'invention se rapporte à une nouvelle méthode *ex vivo* pour évaluer en continu et en temps réel la pression artérielle moyenne d'un patient, notamment d'un patient subissant une anesthésie, qui utilise des valeurs obtenues en temps réel. De préférence, ces valeurs ont été obtenues par pléthysmographie. Cette méthode permet donc à l'anesthésiste de réagir immédiatement en cas de baisse de pression en dessous d'un seuil prédéterminé. Il convient de noter que la méthode décrite ci-après n'est pas mise en oeuvre sur le corps humain, mais est mise en oeuvre *ex vivo,* et utilise des valeurs préalablement mesurées. L'invention n'inclut pas la mesure des valeurs, mais uniquement leur manipulation (décrite ci-après) afin d'obtenir une estimation fiable de la pression artérielle du patient.

Ainsi, les méthodes décrites ci-après se basent préférentiellement sur la mesure de l'onde de pouls par pléthysmographie, qui est une conséquence de l'expulsion systolique, et permettent la déduction et le suivi de la variation de la pression artérielle moyenne, dont on a vu plus haut qu'elle n'est pas liée de façon claire et directe à la pression artérielle systolique. La mesure par pléthysmographie est effectuée à l'aide d'un saturomètre (oxymètre de pouls) au niveau d'un doigt de la main ou du pied, de l'oreille, du front, des ailes du nez, ou d'autre organe ou partie du corps. Il est préféré lorsque cette mesure est effectuée avec un saturomètre au doigt, ou au lobe de l'oreille.

Dans la mesure où la relation entre la pression moyenne et les pressions systolique et diastolique est complexe, et où l'onde de pouls représente la pression systolique et permet de calculer la saturation en oxygène, il est surprenant que l'on puisse utiliser l'onde de pouls pour mesurer la pression moyenne générale. Un tel résultat ne pouvait être envisagé à la vue de l'art antérieur, et l'on peut noter que les documents mentionnés ci-dessus n'en parlent pas, alors même que la pression moyenne est le paramètre qui a du sens en anesthésie pour vérifier une bonne perfusion des organes.

L'invention est définie dans les revendications , et se rapporte ainsi à une méthode (ou un procédé) réalisé *ex vivo* pour évaluer en continu la pression artérielle moyenne d'un patient, sur la base de valeurs d'un paramètre mesuré en continu, comprenant les étapes :
I. Calculer une valeur de calibration *Calib* à partir
   a. De la valeur de la pression artérielle à un temps t0
   b. De la valeur Vp0 liée à la mesure du paramètre obtenue chez le patient au temps t0,
II. Calculer la valeur estimée PAMest de la pression artérielle du patient à un temps t postérieur à t0 par la formule PAMest = k x Calib x Vpt, dans laquelle Vpt est la valeur de la mesure du paramètre obtenue au temps t.

Cette méthode fournit donc une valeur de la pression artérielle moyenne à chaque battement de cœur. Cette méthode est très favorablement mise en oeuvre par ordinateur, donc *in silico.*

Ainsi, on peut évaluer la pression artérielle moyenne en continu en utilisant le coefficient de corrélation *Calib* calculé sur la base d'une mesure réelle de la pression artérielle moyenne au temps t0 et d'une mesure du paramètre physiologique que l'on peut obtenir en continu au même temps t0. La pression artérielle moyenne (à t0 ou ultérieurement en cas de recalibration) est mesurée par toute méthode connue dans l'art (brassard ou directement par sonde intraartérielle). On peut recalculer la valeur Calib de temps à autre, en particulier à intervalles réguliers par mesure d'une nouvelle valeur de la pression artérielle et mesure d'une nouvelle valeur du paramètre Vp, et utiliser cette nouvelle valeur Calib recalculée ultérieurement, jusqu'à la nouvelle calibration.

Il est préféré quand le paramètre *Vp* mesuré en continu l'est par pléthysmographie. Il existe diverses méthodes de pléthysmographie, et la méthode préférée dans le cadre de l'invention est la photopléthysmographie ou pléthysmographie par effet photoélectrique.

Cette méthode permet de déterminer le sur-volume de sang à chaque battement de cœur (« PI » pour *« perfusion index »* ou indice de perfusion) lié à l'expulsion sanguine après systole. Ainsi, le PI s'exprime en pourcentage par rapport au volume sanguin « non pulsatile » du doigt.

Cette méthode permet également de mesurer le rapport entre la désoxy- et l'oxyhémoglobine (connu sous le terme SpO₂).

Le rendu d'une photopléthysmographie est un graphe représentant la variation du volume mesuré, ainsi que les deux valeurs PI et SpO₂ mentionnées ci-dessus. Ce graphe représente l'onde de pouls et reflète l'expulsion sanguine au moment de la systole. On peut ainsi y repérer les deux pics de pression qui envoient le sang dans l'organisme (pic principal à la sortie du cœur et pic secondaire (onde dicrote) après relaxation des vaisseaux proches du ventricule). Cette onde dicrote peut être représentée, sur le graphe, par un second pic, apparaissant au cours de la décroissance observée pour le pic principal, par un plateau, ou par une variation de pente lors de la décroissance du pic principal (correspondant à l'afflux de sang, qui est trop faible toutefois pour créer un nouveau pic ou un plateau). La forme prise de cette seconde onde de pression (onde dicrote), au niveau du graphe du pléthysmographe, dépend de plusieurs facteurs, tels la nature et précision du pléthysmographe, de l'état cardiaque du patient, et/ou de l'état des vaisseaux du patient. Toutefois, ainsi que mentionné plus haut, il est toujours possible de détecter cette deuxième onde de pression.

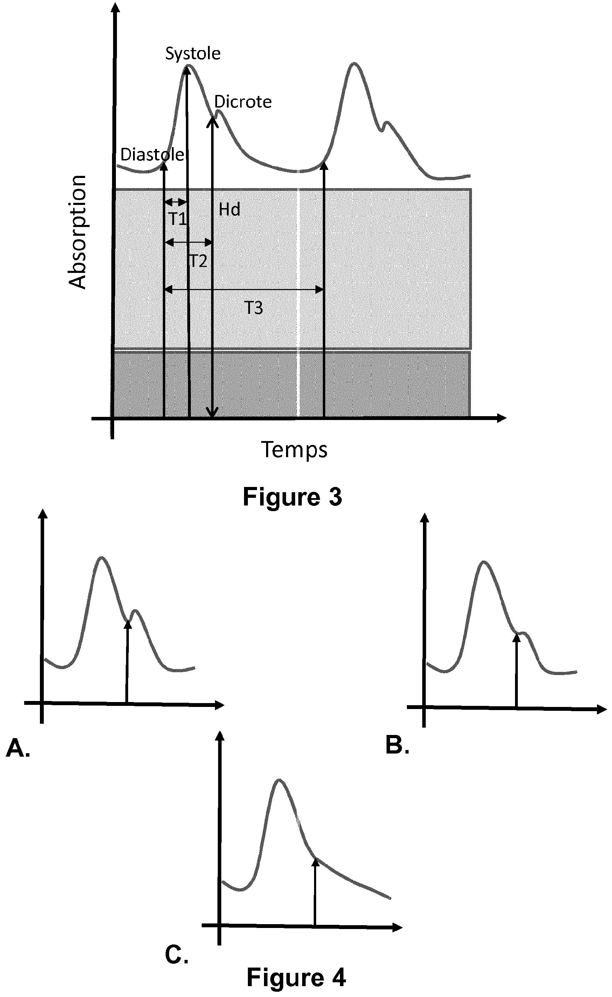

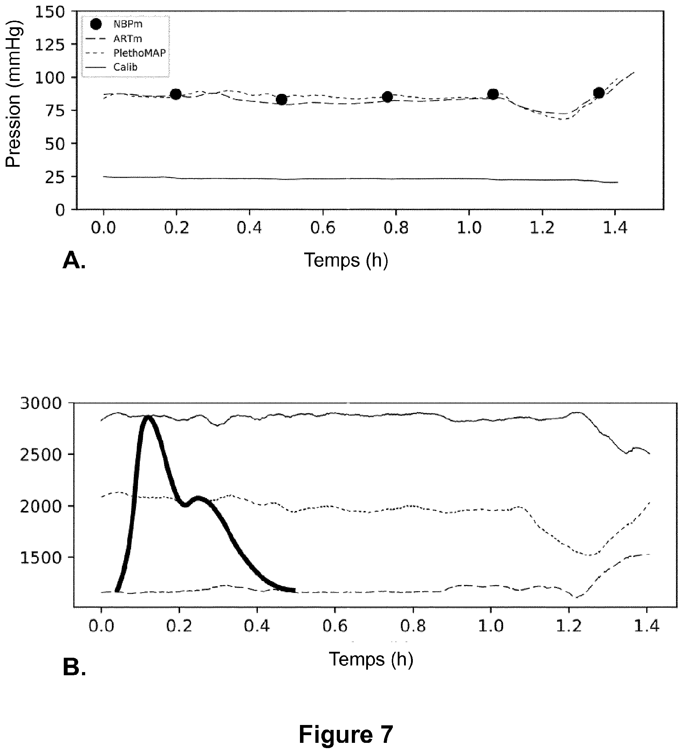

Dans l'invention, on utilise, en tant que valeur Vpt du paramètre mesuré en continu, la valeur de l'onde dicrote. On utilise ainsi la hauteur de cette onde dicrote, si on peut observer un pic ou un plateau (figure 4.A et 4.B. Si l'on observe uniquement une rupture dans la courbe de décroissance, on utilise, comme début de l'onde dicrote, l'endroit de cette rupture (figure 4.C). La hauteur de l'onde dicrote (encoche dicrote) utilisée est très préférentiellement la hauteur totale ou hauteur absolue du signal de pléthysmographie (Hd, figure 3) de préférence à la hauteur mesurée par rapport à la ligne de base de la partie pulsatile (qui correspond à la hauteur diastolique). De fait, la valeur d'absorption de cette ligne de base est susceptible de varier au cours du temps (figure 7.B), ce qui peut mener à des mauvaises évaluations si l'on ne prend pas en compte la valeur absolue de l'absorption mesurée à l'apparition de l'onde dicrote.

Dans un autre mode de réalisation, la valeur Vpt que l'on peut calculer en continu est liée au logarithme de l'inverse de l'indice de perfusion (PI) au temps t.

Dans un autre mode de réalisation, la valeur Vpt utilisée est la durée entre le début de l'onde pouls et l'onde dicrote (la rupture de pente observée lors de la décroissance de l'onde de pouls) désignée en tant que T2 dans la figure 3.

Dans un autre mode de réalisation, la valeur Vpt utilisée est la durée entre le début de l'onde pouls et le maximum de l'onde de pouls, désignée en tant que T1 dans la figure 3.

Dans un autre mode de réalisation, la valeur Vpt utilisée est la période de l'onde pouls (durée mesurée entre les pieds de deux odes de pouls successives), désignée en tant que T3 dans la figure 3. Ce paramètre est essentiellement utilisé en tant que paramètre secondaire, lorsque l'on utilise plusieurs variables, notamment pour pondérer la PAMest calculée avec un autre paramètre.

Dans un autre mode de réalisation, la valeur Vpt est le ratio de la valeur de l'onde dicrote par rapport à la valeur du pic maximal systolique ou le ratio de la valeur de l'onde dicrote par rapport à la valeur du de la diastole (pied de l'onde de pouls).

Ainsi que mentionné plus haut, l'onde de pouls est l'onde de pression que l'on peut détecter suite à l'afflux de sang au niveau d'un organe, et qui est donc liée aux battements cardiaques et à la systole.

Il est très intéressant d'utiliser la hauteur de l'onde dicrote, qui permet d'estimer la valeur de la PAM. Toutefois, il peut être intéressant d'utiliser également l'indice de perfusion (directement, son inverse ou le logarithme de son inverse). En effet, l'indice de perfusion varie de façon inverse à la PAM (voir les exemples), c'est-à-dire qu'il augmente lorsque la PAM diminue. On voit également que l'indice de perfusion est un marqueur très sensible, qui commence à « bouger » (augmenter) dès que la PAM diminue, cette variation étant plus précoce que celle de la hauteur de l'onde dicrote. Ainsi, on peut envisager de surveiller les deux marqueurs (hauteur de l'onde dicrote et indice de perfusion), en étant attentif en cas d'augmentation de l'indice de perfusion (ou de diminution de l'inverse de l'indice de perfusion ou du logarithme de cette inverse) et encore plus attentif si cette augmentation est suivie d'une diminution de la hauteur de l'onde dicrote. Ainsi, l'invention se rapporte également à une méthode *(ex vivo)* d'évaluation ou d'estimation de la pression artérielle moyenne d'un patient, comprenant les étapes de
- Mesurer la valeur de l'indice de perfusion, notamment par photopléthysmographie
- Évaluer la variation de cet indice ou d'une fonction de cet indice (telle que l'inverse de l'indice, logarithme de l'inverse de l'indice)
- Évaluer la valeur de la pression artérielle moyenne par une méthode telle que décrite ci-après (notamment *via* la mesure de la hauteur de l'onde dicrote par photopléthysmographie) en cas de variation de l'indice de perfusion (augmentation) ou de la variable composée (inverse de l'indice, logarithme de l'inverse de l'indice, diminution).

L'invention se rapporte aussi à une méthode (ou un procédé) pour évaluer en continu la pression artérielle moyenne d'un patient, sur la base de valeurs d'un paramètre mesuré en continu, comprenant les étapes :
I. Mesurer la pression artérielle moyenne chez un patient à un temps t0
II. Mesurer la valeur du paramètre Vp0 au temps t0
III. Calculer une valeur de calibration Calib à partir
   a. De la valeur de la pression artérielle obtenue en I au temps t0
   b. De la valeur Vp0 obtenue en II au temps t0,
IV. Mesurer la valeur du paramètre Vpt au temps t postérieur à t0
V. Calculer la valeur estimée PAMest de la pression artérielle du patient au temps t par la formule PAMest = k x Calib x Vpt, dans laquelle Vpt est la valeur de la mesure du paramètre obtenue en V.

On peut aussi effectuer une recalibration de temps en temps (par exemple toutes les 5 minutes). Cette recalibration consiste en un nouveau calcul de la valeur Calib de façon régulière et l'utilisation de cette nouvelle valeur Calib jusqu'à la recalibration suivante.

L'invention se rapporte à une demande de traitement d'un patient qui en a besoin, comprenant l'étape d'administration d'un vasopresseur au patient, lorsque la pression artérielle moyenne estimée (PAMest), telle que calculée par les méthodes décrites dans la présente demande, baisse ou descend en dessous d'un seuil prédéterminé. Ceci indique alors que le patient est en hypotension et l'administration d'une quantité thérapeutiquement efficace d'un vasopresseur (qui permet d'augmenter une pression artérielle diminuée) permettra de rétablir la pression artérielle adéquate. Les vasopresseurs sont connus dans l'art et incluent les sympathicomimétiques (dont l'adrénaline, la dopamine, l'éphédrine...), les glucocorticoïdes et mineralocortïcoides, l'angiotensinamide... En milieu hospitalier, on utilise plutôt des sympathicomimétiques.

Dans un mode de réalisation particulier, la valeur Vpt utilisée à l'instant t est une valeur moyennée de plusieurs valeurs mesurées pendant une durée prédéterminée. Utiliser une telle valeur moyennée permet de s'affranchir d'une éventuelle variation singulière à un instant t. On peut notamment utiliser la valeur moyennée sur trois systoles.

Dans un mode de réalisation particulier, la méthode décrite ci-dessus est mise en oeuvre sur un patient durant une anesthésie générale. Cette méthode est donc mise en oeuvre pendant une durée de de plusieurs dizaines de minutes, voire plusieurs heures. Il peut être opportun d'effectuer une nouvelle calibration de temps en temps, notamment lors de la prise de la tension artérielle au brassard.

Ainsi, la méthode décrite ci-dessus (mesure réelle de la pression artérielle, mesure du paramètre, calcul de la valeur *Calib,* évaluation ultérieure de la pression artérielle en utilisant cette valeur *Calib)* peut être répétée plusieurs fois à des intervalles prédéterminés. En particulier, on peut répéter cette méthode à chaque fois que l'on prend la tension au brassard (c'est-à-dire essentiellement recalculer une valeur *Calib),* ou toutes les deux fois, ou toutes les trois fois. Ceci permet d'éviter une éventuelle dérive de la valeur estimée de la pression artérielle moyenne entre deux mesures réelles. On peut aussi effectuer ce calcul de la valeur *Calib* si la valeur estimée de la pression artérielle moyenne est trop éloignée (variation de 10 % ou de 5 %) de la valeur réelle mesurée de temps en temps.

Dans un autre mode de réalisation, on peut également affiner la valeur PAMest évaluée au temps t, en calculant plusieurs de ces valeurs (en utilisant différents paramètres mesurables en continu) et en effectuant une interpolation (évaluation) de la valeur PAMest réelle, en fonction des plusieurs résultats obtenus. Ainsi, on fait n mesures individuelles avec n paramètres (par exemple onde dicrote, PI, différence de temps entre les pics...) puis on mesure la PAMest estimée pour chacun des paramètres, et on calcule une PAMest finale, de façon probabilistique.

On décrit ainsi une méthode *ex vivo* pour évaluer en continu la pression artérielle moyenne chez un patient, caractérisée en ce que
a. on répète la méthode décrite ci-dessus pour différents paramètres, afin d'obtenir plusieurs valeurs PAMest au temps t (chacune étant liée à un paramètre particulier)
b. on calcule une valeur PAMestfinale par estimation statistique tenant compte
   i. des différentes valeurs PAMest calculées au temps t
   ii. d'une ou plusieurs valeurs PAMestfinale calculées avant le temps t.

Dans l'étape a), on peut ainsi calculer une PAMest au temps t sur la base des valeurs mesurées des paramètres suivants choisis parmi la hauteur de l'onde dicrote, le logarithme de l'inverse de l'indice de perfusion (PI) (auquel on ajoute 1 pour éviter d'obtenir une valeur négative), la durée entre le début de l'onde pouls et l'onde dicrote, et la durée entre le début de l'onde pouls et le maximum de l'onde de pouls. On peut aussi utiliser le ratio (hauteur de l'onde dicrote / hauteur maximale de l'onde de pouls) et/ou le ratio (hauteur de l'onde dicrote/hauteur de la partie diastolique (pied de l'onde de pouls).

En particulier, on peut ainsi calculer une PAMest au temps t sur la base des combinaisons suivantes des valeurs mesurées des paramètres
- hauteur de l'onde dicrote et logarithme de (l'inverse de l'indice de perfusion (PI) + 1)
- hauteur de l'onde dicrote et durée entre le début de l'onde pouls et l'onde dicrote
- hauteur de l'onde dicrote et durée entre le début de l'onde pouls et le maximum de l'onde de pouls
- hauteur de l'onde dicrote, logarithme de (l'inverse de l'indice de perfusion (Pl) + 1), et durée entre le début de l'onde pouls et l'onde dicrote
- hauteur de l'onde dicrote, logarithme de (l'inverse de l'indice de perfusion (Pl) + 1), et durée entre le début de l'onde pouls et le maximum de l'onde de pouls
- hauteur de l'onde dicrote, durée entre le début de l'onde pouls et le maximum de l'onde de pouls, et durée entre le début de l'onde pouls et l'onde dicrote
- hauteur de l'onde dicrote, logarithme de (l'inverse de l'indice de perfusion (PI) + 1), durée entre le début de l'onde pouls et l'onde dicrote, et durée entre le début de l'onde pouls et le maximum de l'onde de pouls

D'autres combinaisons n'incluant pas la hauteur de l'onde dicrote peuvent également être envisagées et /ou intégrant les ratios (hauteur de l'onde dicrote / hauteur maximale de l'onde de pouls) et/ou (hauteur de l'onde dicrote/hauteur de la partie diastolique).

Il est préféré quand la hauteur de l'onde dicrote (ou le ratio hauteur de l'onde dicrote / hauteur de la systole, ou le ratio hauteur de l'onde dicrote / hauteur de la systole) fait partie des variables mesurées et utilisées, les autres variables étant choisies parmi celles mentionnées ci-dessus. De fait, et ainsi que montré dans les exemples, les paramètres liés à l'onde dicrote (et en particulier à sa hauteur) permettent d'obtenir une très bonne valeur de la pression artérielle moyenne, et auront essentiellement le poids le plus important dans la détermination de la PAMestfinale, les autres variables servant essentiellement à pondérer les variables liées à l'onde dicrote ce qui peut s'avérer intéressant pour certains patients.

À l'issue de la mise en oeuvre de l'étape a), on obtient ainsi n valeurs estimées de la pression artérielle moyenne (n étant le nombre de paramètres sélectionnés).

L'étape b) consiste à évaluer une pression artérielle moyenne sur la base de ces n valeurs estimées, et de la valeur estimée au temps précédent.

Une telle évaluation peut être effectuée par toute méthode statistique connue dans l'art, et en particulier en utilisant un filtre de Kalman en contexte discret. Le filtre de Kalman en contexte discret est un estimateur récursif. Cela signifie que pour estimer l'état à un temps t, on utilise uniquement l'estimation de l'état précédent et les mesures au temps t. L'historique des observations et des estimations n'est ainsi pas requis.

L'utilisation des valeurs de plusieurs paramètres et de la probabilité statistique ainsi que proposée par des filtres tels que le filtre de Kalman permet d'augmenter la fiabilité de la mesure affichée de la pression artérielle moyenne, par rapport à une mesure évaluée uniquement sur la base d'un seul paramètre. Cela permet notamment de ne pas donner trop de poids à des mesures aberrantes que l'on pourrait obtenir pour un paramètre à un moment donné (quelle qu'en soit la raison).

Dans un autre mode de réalisation, on décrit ainsi une méthode ex vivo pour évaluer en continu la pression artérielle moyenne chez un patient, caractérisée en ce que
a. on répète la méthode décrite ci-dessus pour différents paramètres (Vpnt), afin d'obtenir plusieurs valeurs PAMest (PAMestn) au temps t (chacune étant liée à un paramètre particulier)
b. on calcule une valeur PAMestfinale par combinaison des différentes valeurs PAMest calculées au temps t

La combinaison décrite à l'étape b) est préférentiellement une régression linéaire, et la PAMestfinale s'écrit a1PAMest1 + a2 PAMest2 + ... (PAMestn correspondant à la valeur PAMest obtenue pour le paramètre Vpnt mesuré au temps t.

Cette régression linéaire est effectuée par toute méthode connue dans l'art, en tenant compte du poids relatif des valeurs PAMestn pour chaque paramètre. Les facteurs a1, a2... sont préférentiellement recalculés à chaque mesure réelle de pression moyenne au brassard.

De préférence, on utilise cette méthode lorsque l'on a déjà un certain nombre de données, ce qui permet d'affiner la qualité des coefficients. On peut ainsi utiliser, comme coefficients initiaux, des coefficients préalablement calculés sur une cohorte d'autres patients (au moins 50, préférablement au moins 100 patients). En effet, même s'il existe une variabilité inter-patients et que les coefficients obtenus sur cette cohorte ne sont pas nécessairement les meilleurs pour le patient en question, ces coefficients préalablement calculés peuvent être utilisés avant d'être affinés en fonction des données obtenues pour le patient. Ainsi, lors de chaque calibration,
- on calcule la PAMest avec les coefficients précédemment utilisés (les coefficients initiaux (provenant de la cohorte) lors de la première calibration)
- on compare cette valeur à la valeur de la PAM mesurée
- on réajuste les coefficients en donnant de plus en plus de poids aux valeurs mesurées pour le patient, dans la régression au fur et à mesure que l'on dispose des données de PAM mesurée chez ce patient.

Ainsi, les méthodes décrites ci-dessus sont basées sur le fait que la pression artérielle moyenne peut être mesurée en fonction de paramètres que l'on peut mesurer en continu, préférentiellement à partir d'une mesure de pléthysmographie. En particulier, la hauteur de l'onde dicrote (ou le ratio par rapport à la valeur totale de l'onde systolique et/ou l'onde diastolique) ou le logarithme de l'inverse de l'indice de perfusion (augmenté de 1) sont proportionnels à la pression artérielle moyenne. La méthode pour obtenir la pression artérielle moyenne est donc beaucoup plus simple que celles décrites dans l'art antérieur, tout en étant fiable.

Ainsi que mentionné plus haut, les méthodes décrites sont particulièrement intéressantes pour suivre l'état de la pression artérielle d'un patient en continu lorsque celui-ci est en état d'anesthésie générale. Cela permet ainsi au médecin de pouvoir agir rapidement en cas de de pression trop faible, sana avoir à attendre la mesure réelle obtenue au brassard.

Ainsi, il est préféré lorsque ces méthodes sont mises en œuvre en continu pendant toute la durée d'anesthésie générale d'un patient.

Par ailleurs, et afin d'assurer la sécurité du patient, il est possible qu'un signal soit émis lorsque la valeur PAMest est inférieure à un seuil prédéterminé (on peut l'envisager si la pression artérielle moyenne est inférieure à 65 mm Hg). Une telle étape d'émission d'un signal peut être intégrée dans une méthode telle que décrite ci-dessus. Le signal peut être un signal graphique (telle qu'une représentation de la pression artérielle moyenne par un code couleur différent du code couleur classique (rouge en cas d'alerte au lieu de vert ou jaune). On peut aussi envisager l'affichage de la valeur avec différents codes couleurs sur le moniteur de suivi, pour alerter le médecin sur un risque d'hypotension.

Le signal d'alerte émis peut également ou alternativement être un signal sonore (bip long ou autre) lorsque la pression artérielle moyenne devient inférieure à une valeur prédéterminée. Ceci permet également d'alerter le chirurgien d'un problème, et de la nécessité, pour l'anesthésiste d'accomplir l'acte médical permettant de corriger cette hypotension.

L'invention se rapporte également à un produit/programme d'ordinateur comprenant des instructions de code de programme enregistrées sur un support lisible par un ordinateur, pour mettre en oeuvre les étapes des procédés décrits ci-dessus, lorsque ledit programme est exécuté sur un ordinateur.

Ce programme peut également contenir des instructions de code permettant l'affichage, sur un moniteur, de la valeur de la pression artérielle moyenne. Il peut également inclure des instructions de code permettant l'émission d'un signal visuel et/ou sonore si la valeur de la pression artérielle moyenne estimée est inférieure à un seuil prédéterminé (pré-programmé ou entré par le praticien). Il peut également inclure des instructions de code pour qu'une mesure réelle de la pression artérielle soit effectuée sur le patient si la valeur estimée est inférieure à une valeur prédéterminée. Ainsi, le programme peut demander et commander une prise de pression artérielle avant le temps réglementaires, sans l'intervention d'un être humain.

L'invention se rapporte également à un support d'enregistrement lisible par un ordinateur sur lequel est enregistré un programme d'ordinateur comprenant des instructions de code de programme pour l'exécution des étapes des procédés tels que décrits ci-dessus ou des programmes tels que décrits ci-dessus.

L'invention peut également comprendre un dispositif pour la mise en oeuvre d'une méthode telle que décrite plus haut, comprenant :
- des moyens de réception de données de mesure de la pression artérielle moyenne, en particulier telle que prise par méthode non-invasive (brassard)
- des moyens de réception de données de mesure d'un ou plusieurs paramètres mesurés en continu (notamment la hauteur de l'onde dicrote, ou l'indice de perfusion)
- des moyens de calcul permettant de calculer un coefficient Calib à chaque mesure réelle de pression artérielle moyenne
- des moyens de calcul permettant de calculer une pression artérielle moyenne à chaque instant t en fonction de la valeur des paramètres à cet instant t, selon les méthodes décrites ci-dessus
- des moyens de présentation de la pression artérielle moyenne calculée à chaque instant t (incluant éventuellement des moyens d'alerte dans le cas où la pression artérielle moyenne calculée est inférieure à une valeur prédéterminée)
- éventuellement des moyens permettant l'actionnement automatique du dispositif pour mesurer la pression artérielle moyenne, dans le cas où la pression artérielle moyenne calculée est inférieure à une valeur prédéterminée
- éventuellement des moyens permettant l'administration automatique d'une dose de vasopresseur, dans le cas où la pression artérielle moyenne calculée ou estimée est inférieure à une valeur prédéterminée.

Les moyens de calcul sont essentiellement des processeurs permettant l'exécution des produits/programmes d'ordinateur tels que mentionnés ci-dessus. Les moyens de réception de données, de présentation de la pression artérielle moyenne, d'actionnement du sphygmomanomètre ou d'administration automatique d'une dose de vasopresseur sont des moyens classiques dans l'art.

Les méthodes décrites ci-dessus sont de premier intérêt dans le domaine de l'anesthésie et salle de surveillance post-interventionelle, mais sont aussi utilisables dans d'autres domaines tels que la réanimation (en particulier les patients hyperventilés), la cardiologie, la médecine de ville, ou la médecine d'urgence (préhospitalier et interhospitalier). On peut aussi utiliser les méthodes et dispositifs dans la médecine du sport. On peut aussi utiliser les méthodes et dispositifs pour évaluer la pression artérielle moyenne dans des tests d'effort chez un patient.

L'invention se rapporte également à une méthode d'analyse d'un signal de pléthysmographie, et notamment à une ou des méthodes pour déterminer le pied de l'onde de pouls, le maximum de l'onde de pouls, et/ou l'onde dicrote, sur un tracé de pléthysmographie, en appliquant les méthodes décrites plus précisément dans les exemples.

En particulier, on détermine le pied de l'onde de pouls en calculant la seconde dérivée du signal de pléthysmographie, le pied de l'onde de pouls correspondant au maximum de cette seconde dérivée, au front ascendant de l'onde de pouls. On peut pondérer par la première dérivée pour s'intéresser uniquement à la partie croissante du signal (c'est-à-dire que l'on donne une valeur nulle à la seconde dérivée lorsque la première dérivée n'est pas positive). On peut aussi mettre les valeurs obtenues au carré puis les intégrer de manière flottante sur des fenêtres de temps prédéterminé, en particulier 240ms centrées (moyenne sur les valeurs 120 ms avant et 120 ms après le point désiré), afin d'obtenir un signal puissant à chaque front ascendant de l'onde de pouls. On peut comparer ce signal à une valeur seuil. De préférence, cette valeur seuil est adaptative en temps réel. La valeur seuil peut être calculée par la formule : l'intégrale (telle que calculée ci-dessus) moyennée sur une fenêtre flottante de 3s (centrée ou pas), et dont la valeur est multipliée par 1,5. On identifie le pic (maximum) de la seconde dérivée (qui définit le pied de l'onde) dans la zone où l'intégrale dépasse cette valeur seuil.

Le pic (maximum) de l'onde de pouls peut être déterminé, après le pied de l'onde de pouls en
- Découpant le signal en plusieurs fenêtres de temps (en particulier des fenêtres entre 20 ms et 100 ms, en particulier de 50 ms), et
- regardant la valeur maximale dans chaque fenêtre de temps (on avance fenêtre par fenêtre tant que des valeurs plus élevées sont trouvées dans la dernière fenêtre de temps étudiée).

La valeur maximale locale (correspondant à la valeur V maximale trouvée dans une fenêtre de temps, la valeur maximale de la fenêtre de temps suivant étant inférieur à cette valeur V) correspond au maximum de l'onde de pouls.

Cette méthode permet d'obtenir
- la valeur du maximum (valeur en absorbance donnée par l'oxymètre de pouls correspondant au pic de l'onde systolique)
- le moment auquel ce maximum est atteint.

La valeur et le moment de l'onde dicrote peuvent être déterminés par
- obtention de la seconde dérivée du signal après le maximum de l'onde de pouls
- découpage du signal sur des fenêtres de temps prédéterminées (entre 50 ms et 300 ms, en particulier de 150 ms) après ce pic de l'onde de pouls
- recherche du maximum local de la seconde dérivée du signal dans chaque fenêtre de temps, afin de déterminer une zone d'intérêt (fenêtre de temps dans laquelle on a ce maximum local de la seconde dérivée du signal)
- Recherche du minimum en valeur absolue de la première dérivée du signal dans cette zone d'intérêt. On peut de nouveau effectuer cette recherche découpant cette zone d'intérêt en fenêtre de temps (entre 5 ms et 15 ms, en particulier 8 ms).

Le point correspondant à l'onde dicrote correspond à ce point pour lequel on atteint le minimum en valeur absolue de la première dérivée. On peut alors mesurer sa valeur (valeur en absorbance donnée par l'oxymètre de pouls), ainsi que la durée entre le pied de l'onde de pouls et ce point.

### BREVES DESCRIPTION DES FIGURES

Figure 1 : exemple de graphe d'un signal de sphygmomanomètre (obtenu du site D'après https://www.infirmiers.com/etudiants-en-ifsi/cours/cours-cardiologie-la-pression-arterielle-et-sa-mesure.html)
Figure 2 : Principe de l'oxymétrie de pouls. (1) : absorption lumineuse variable liée à la variation du volume de sang artériel. (2) absorption lumineuse constante liée à la partie non pulsatile du sang artériel. (3) : absorption lumineuse constante liée au sang veineux. (4) : absorption lumineuse constante lié aux tissus, os... D'après Feissel, Réanimation 16 (2007) 124-131.
Figure 3 : Représentation des variables utilisables dans le cadre de l'invention, à partir d'un tracé d'oxymètre de pouls. T1 : durée entre le début et le maximum de l'onde de pouls ; T2 : durée entre le début de l'onde de pouls et l'onde dicrote ; T3 : durée totale de l'onde de pouls ; Hd : hauteur de l'onde dicrote.
Figure 4 : représentations de différents types de signaux de Pléthysmographie avec repérage de l'onde dicrote.

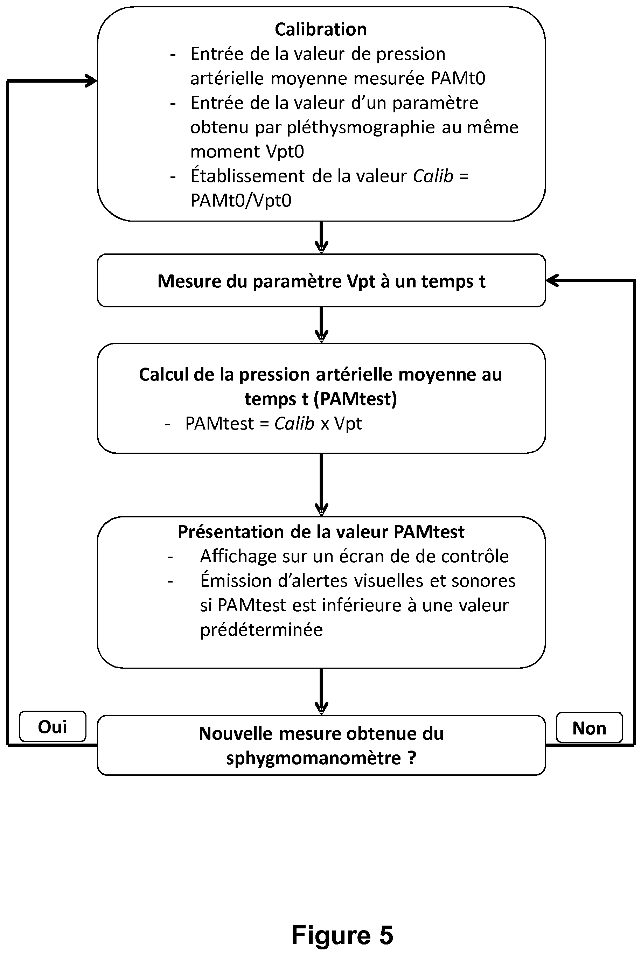

Figure 5 : logigramme représentant la mise en œuvre d'un procédé selon l'invention.

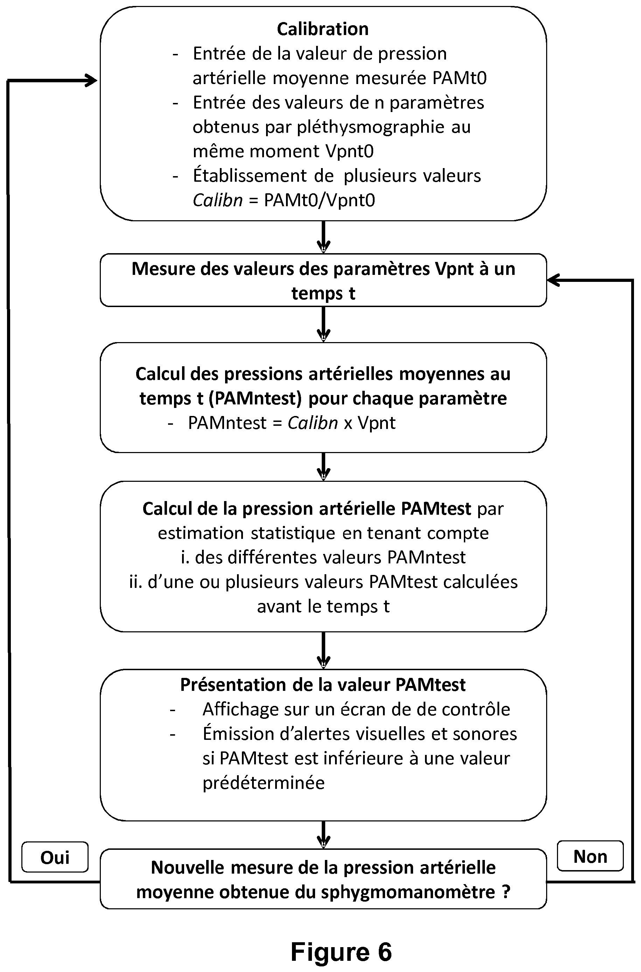

Figure 6 : logigramme représentant la mise en œuvre d'un autre mode de réalisation d'un procédé selon l'invention.
Figure 7 : A. Représentation, chez un patient représentatif, de la PAM mesurée par méthode invasive (ARTm, grands pointillés), la PAM estimée par une méthode selon l'invention sur la base de la hauteur de l'onde dicrote (PlethoMAP, petits pointillés), la PAM mesurée par le sphygmomanomètre brassard (NBPm, point noirs) et le facteur de calibration (Calib, ligne continue). B. Représentation, pour le même patient et la même période, l'évolution du pic systolique (ligne continue), de la dicrote (petits pointillés) et de la diastole (grands pointillés) au cours du temps. Une représentation graphique de l'onde de pouls superposée est également indiquée sur cette figure, uniquement pour améliorer la compréhension des trois points (ce à quoi correspond chaque valeur). À noter : les échelles de temps des cette représentation de l'onde de pouls et de l'évolution des pics au cours du temps sont différentes et cette représentation de l'onde de pouls (durant généralement 1 sec ou moins) est uniquement présente d'un point de vue informatif.

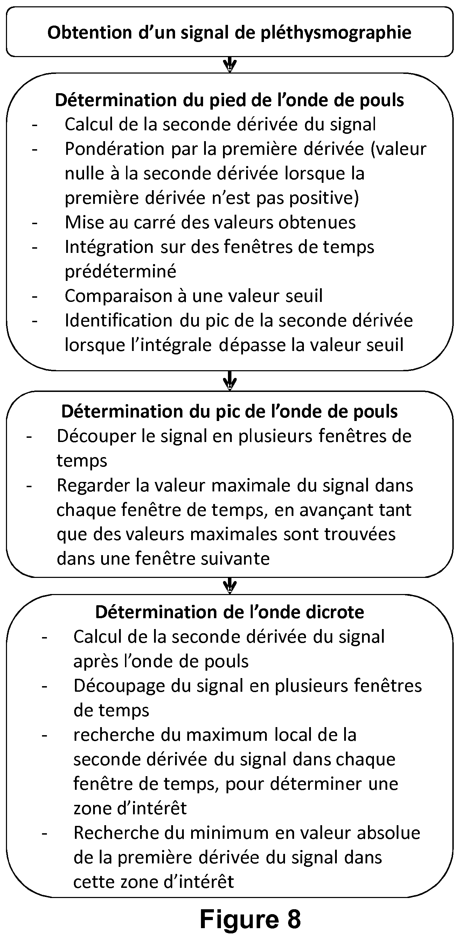

Figure 8 : logigramme représentant la mise en œuvre d'un mode de réalisation d'analyse du signal de pléthysmographie selon un procédé selon l'invention.
Figure 9 : Définition des Dicpleth et PI. a = amplitude de la composante pulsatile du signal photopléthysmographique (hauteur du pic systolique, partie pulsatile) ; b= hauteur de l'encoche dicrotique (partie pulsatile) ; c = amplitude de la composante stationnaire du signal photopléthysmographique.

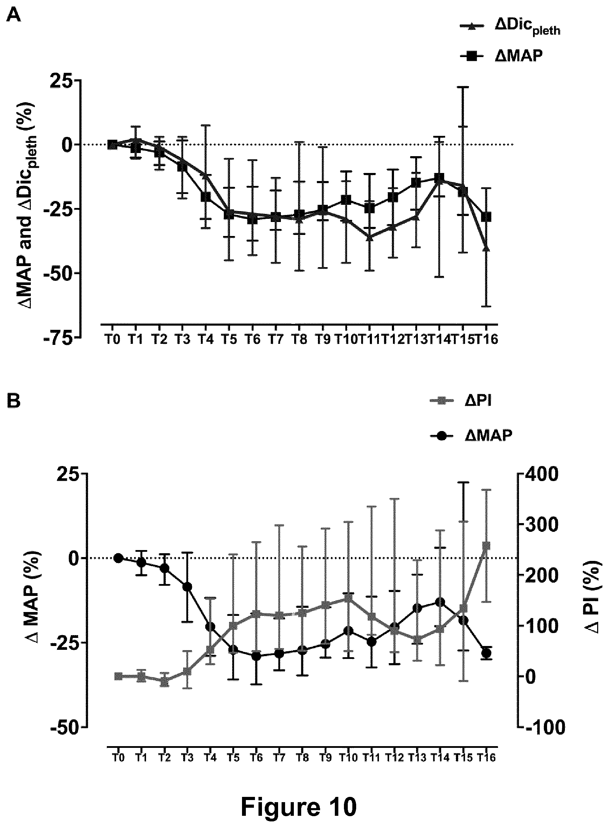

Figure 10 : Valeurs médianes de ΔPAM et ΔDicpleth (A) et ΔPAM et ΔPI (B) pendant l'induction de l'anesthésie. ΔDicpleth : variation relative de Dicpleth par rapport à la ligne de base ; ΔPI : variation relative de l'indice de perfusion par rapport à la ligne de base ; ΔPAM : variation relative de la PAM par rapport à la ligne de base. La figure représente l'évolution de ΔPAM et ΔDicpleth pendant les 16 min d'induction de l'anesthésie. La PAM médiane et la variation de la Dicpleth par rapport à la ligne de base sont représentées à chaque minute (T "x", à "x" min à partir de T0) à partir du début de l'induction (T0).

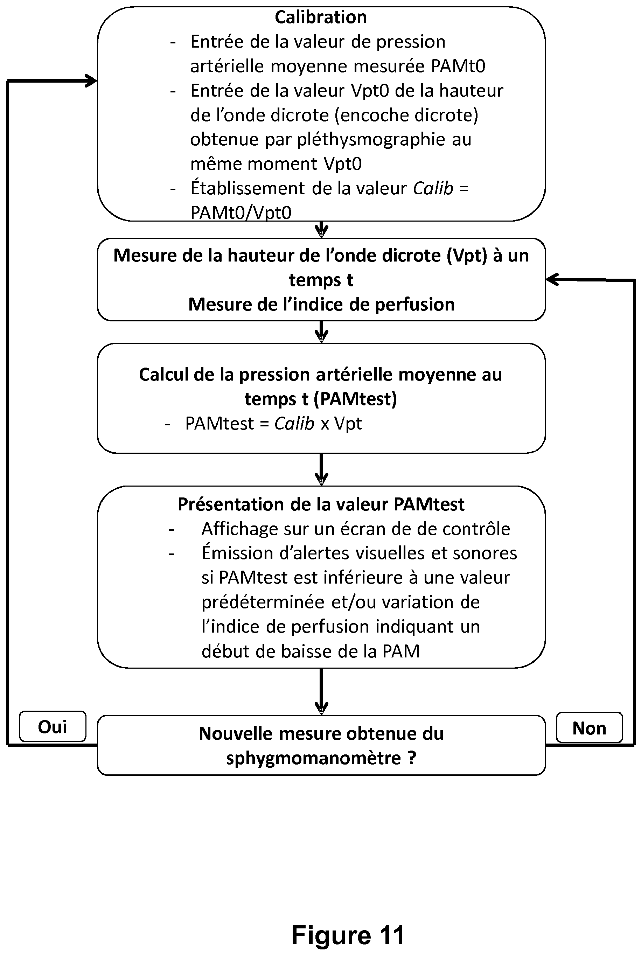

Figure 11 : logigramme représentant la mise en œuvre d'un procédé selon l'invention, utilisant la mesure de la hauteur de l'encoche dicrote, et de l'indice de perfusion.

### EXEMPLES

Les exemples ci-dessous illustrent différents aspects et modes de mise en oeuvre de l'invention. Les modes de réalisations décrits dans les exemples sont parties intégrantes de l'invention.

### Exemple 1. Détermination des paramètres utilisables pour mesurer la pression artérielle en continu

En cardiologie, il est ainsi possible de mesurer la variation de l'onde de pouls de manière non invasive par pléthysmographie. On obtient ainsi une courbe (graphe) représentant l'excès volumique dû à l'expulsion systolique.

L'indice de perfusion (PI) reflète la quantité de flux sanguin mesurée localement et en partie du flux artériel pulsé, du volume d'éjection systolique. L'indice de perfusion représente l'aire sous la courbe mentionnée ci-dessus. Dans le cas d'une pléthysmographie par effet photoélectrique, on obtient également une valeur SpO₂, représentant la saturation pulsée en oxygène.

La courbe représente le profil de l'onde de pouls et permet de voir l'onde dicrote en sortie de cœur à la systole, (un second pic (éventuellement deux pics), un plateau ou une rupture de la décroissance) dans l'organe.

Ce signal de pléthysmographie peut être utilisé pour la mesure de la pression artérielle moyenne en continu.

On utilise ainsi les variations de taille de pics (de l'onde dicrote), d'aire (valeur de l'indice de perfusion) ou de timing entre deux événements du profil de l'onde de pouls. On peut notamment effectivement voir la décroissance de l'onde dicrote, c'est-à-dire que l'on peut détecter cette onde dicrote de façon certaine dans le signal de pléthysmographie.

Afin de déterminer ces paramètres, il est possible d'analyser l'onde de pouls.

### Pied de l'onde de pouls

On peut, dans un premier temps, détecter le pied de l'onde de pouls. Le pied de l'onde de pouls est caractérisé par une montée rapide du signal, ce qui se traduit par un pic de la seconde dérivée du signal. L'analyse de cette seconde dérivée du signal obtenu par le pléthysmographe permet d'obtenir un signal (pic de la seconde dérivée) à chaque front ascendant de l'onde de pouls, et donc de détecter le pied de l'onde de pouls, et donc le moment correspondant au début du signal.

### Systole (pic de l'onde de pouls)

À partir du pied de l'onde, pour chaque cycle, on cherche la valeur maximale du cycle. Pour cela, on peut découper le signal en plusieurs fenêtres de temps et regarder la valeur maximale dans chaque fenêtre de temps. On peut ainsi déterminer la valeur maximale locale correspondant au maximum de l'onde de pouls. On obtient ainsi
- la valeur du maximum (valeur en absorbance donnée par l'oxymètre de pouls correspondant au pic de l'onde systolique)
- le moment auquel ce maximum est atteint

On peut donc calculer la durée entre le début de l'onde de pouls et le maximum de l'onde de pouls.

### Onde dicrote

Une fois que le pic de l'onde de pouls a été identifié, la seconde dérivée du signal est analysée sur des fenêtres de temps prédéterminées (entre 50 ms et 300 ms). On recherche le maximum local de la seconde dérivée qui se situe après le pic systolique, afin de déterminer une zone d'intérêt dans laquelle le minimum en valeur absolue de la première dérivée est recherché. Le point point dicrotique correspondant à l'onde dicrote correspond à ce point pour lequel on atteint le minimum en valeur absolue de la première dérivée. On peut alors mesurer sa valeur (valeur en absorbance donnée par l'oxymètre de pouls), ainsi que la durée entre le pied de l'onde de pouls et ce point.

### Exemplification d'application pratique de cette méthode

### Recueil du signal

Le recueil du signal a été effectué en temps réel depuis un moniteur patient standard capable de fournir une onde de photopléthysmographie ainsi que la pression artérielle non invasive par brassard à tension. La connexion au moniteur s'effectue habituellement via un port série RS232 ou une connexion réseau via Ethernet ou WIFI. En plus de ces deux paramètres essentiels, on a utilisé également la valeur de l'index de perfusion (PI). La communication avec le moniteur peut être bidirectionnelle et permettre par exemple de demander une nouvelle prise de pression non invasive à la demande.

### Analyse du signal

Le traitement du signal repose sur un algorithme online qui les valeurs mesurées et produit un résultat battement par battement en temps réel.

### Pied de l'onde de pouls

Le logiciel utilise un algorithme de détection de battement cardiaque qui repose sur la détection du pied de l'onde de pouls. Le pied de l'onde de pouls est caractérisé par une montée rapide du signal, ce qui se traduit par un pic de la seconde dérivée du signal.

La détection du pied de l'onde de pouls est basée sur la seconde dérivée du signal. Cette seconde dérivée est pondérée par la première dérivée pour s'intéresser uniquement à la partie croissante du signal (c'est-à-dire que l'on donne une valeur nulle à la seconde dérivée lorsque la première dérivée n'est pas positive). Les valeurs obtenues sont mises au carré puis intégrées de manière flottante sur une fenêtre de 240ms centrée (moyenne sur valeurs 120 ms avant et 120 ms après le point désiré). Ce signal intégré permet d'obtenir un signal puissant à chaque front ascendant de l'onde de pouls.

Ce signal est comparé à une valeur seuil. Cette valeur seuil dépend du patient, du matériel utilisé, de la forme du signal de pléthysmographie ainsi que du bruit de mesure. Le bruit de mesure n'étant pas constant, le seuil est nécessairement adaptatif en temps réel. Pour calculer le seuil, on utlise l'intégrale (calculée ci-dessus) à laquelle est appliquée une moyenne flottante avec une fenêtre de 3s (centrée ou pas), le résultat étant multiplié par 1,5. Le seuil ainsi obtenu permet de définir une zone d'intérêt où l'intégrale dépasse le seuil. À l'intérieur de cette zone d'intérêt, le pic de la seconde dérivée définit le pied de l'onde.

### Systole (pic de l'onde de pouls)

À partir du pied de l'onde, pour chaque cycle, la détection de l'onde dicrote s'effectue en deux étapes. La première étape consiste à trouver la systole et donc la valeur maximale du cycle. Pour cela, le signal est découpé en fenêtres de 50 ms et le signal est avancé fenêtre par fenêtre tant que des valeurs plus élevées sont trouvées. Une fois que la valeur la plus élevée a été dépassée, la valeur maximale locale (correspondant à la valeur maximale de l'onde de pouls, ou valeur de systole).

### Onde dicrote

Une fois que le pic de l'onde de pouls a été identifié, on progresse à partir de ce point en analysant la seconde dérivée du signal en progressant par des fenêtres de 150ms. On cherche ainsi le maximum local de la seconde dérivée qui se situe après le pic systolique. Une fois ce pic identifié, il informe d'une zone d'intérêt dans laquelle se trouve l'onde dicrote. À partir de ce point, la première dérivée est analysée et le minimum en valeur absolue de la première dérivée est recherché dans une fenêtre de 8ms à partir du pic de la deuxième dérivée.

On obtient ainsi le minimum de la première dérivée proche du pic de la deuxième dérivée. Ce point est défini comme étant le point dicrotique correspondant à l'onde dicrote et sa valeur peut être mesurée (valeur totale en absorbance donnée par l'oxymètre de pouls).

### Exemple 2. Calibration et estimation continue de la pression artérielle moyenne (PAM / MAP)

La calibration nécessite la valeur (Vp) d'au moins un des paramètres suivants
- hauteur de l'onde dicrote
- valeur du logarithme (népérien ou décimal) de l'inverse de l'indice de perfusion PI (In (1/PI + 1)). On ajoute 1 à l'inverse de l'indice de perfusion pour éviter de prendre le logarithme d'une valeur inférieure à 1, et d'obtenir un résultat négatif
- valeur de la durée entre le pied de l'onde de pouls et l'onde dicrote et
- valeur de la durée entre le pied de l'onde de pouls et le maximum de l'onde de pouls
- ratio « hauteur de l'onde dicrote / hauteur de l'onde de pouls » et/ou « hauteur de l'onde dicrote/ hauteur de l'onde en diastole ».
- durée totale de l'onde de pouls

Ces valeurs peuvent être obtenues battement par battement (c'est-à-dire pour chaque onde de pouls). La figure 3 montre la façon dont ces variables sont mesurées.

La calibration nécessite également la valeur de pression artérielle moyenne (PAM) qui peut être obtenue notamment par un brassard à tension non invasif.

On peut utiliser une moyenne sur plusieurs cycles (2, 3, 4 5, 6, 8 ou 10 cycles) de la valeur du paramètre choisi. Ceci permet de s'affranchir de perturbations comme la variabilité respiratoire de la pression et l'irrégularité du cycle cardiaque. Cette moyenne est de préférence la médiane statistique plutôt que la moyenne arithmétique.

Un facteur de calibration *Calib* est estimé lors de la prise de la pression artérielle non invasive et est obtenu par *Calib* = PAM/Vp. On comprend bien que la valeur Calib dépend du paramètre choisi et que la valeur Calib obtenue si l'on choisit la valeur de l'onde dicrote sera différente de la valeur Calib si l'on choisit le logarithme de (l'inverse de l'indice de perfusion (PI) + 1).

Une fois la valeur Calib obtenue, la pression artérielle moyenne est estimée battement par battement en utilisant, comme seul source de données, le signal de photopléthysmographie.

### La PAM estimée (PAMest) au temps t est calculée par la formule

PAMest = *Calib* x VPt, dans laquelle Vpt est la valeur (éventuellement moyennée) du paramètre choisi.

### Exemplification d'application (le paramètre étant l'onde dicrote)

La calibration nécessite les valeurs de l'onde dicrote battement par battement ainsi que des valeurs intermittentes de pression artérielle moyenne par exemple par un brassard à tension non invasif. La valeur de pression dicrotique obtenue par l'onde dicrote est moyennée sur plusieurs cycles.

Le nombre de cycles sur lesquels la valeur est moyennée est réglable (par exemple 5 cycles). Ceci permet de s'affranchir de perturbations comme la variabilité respiratoire de la pression et l'irrégularité du cycle cardiaque. Le moyennage se fait en utilisant la médiane statistique plutôt que la moyenne arithmétique afin d'être plus robuste en présence de bruit. Un facteur de calibration est estimé lors de la prise de la pression artérielle non invasive et se base sur la valeur moyennée actuelle de la dicrote (Pdic) et la pression artérielle moyenne mesurée (PAM). Le facteur de calibration est obtenu par Calib= PAM/Pdic.

### Estimation continue de la pression artérielle moyenne (PAM / MAP)

Une fois que la calibration est effectuée, la pression artérielle moyenne est estimée battement par battement en utilisant comme seul source de signal l'absorption mesurée par photoplethysmographie. La PAM estimée (PAMest) est calculée par PAMest= Calib·Pdic, où Pdic est valeur moyennée de la valeur de l'onde dicrote comme décrit plus haut.

### Autre exemple (Utilisation de l'indice de perfusion), notamment en tant que qualité du signal

La valeur du PI sert d'indicateur de la qualité du signal. En effet, une valeur de PI inférieure à 0.1% indique un mauvais signal de photoplethysmographie et permet d'indiquer à l'utilisateur une qualité d'estimation médiocre et la nécessité plus fréquente d'une calibration. La qualité du signal peut en général être améliorée dans ces cas en repositionnant correctement le capteur sur le patient.

### Intégration dans l'estimation

Le PI donne des informations sur l'état hémodynamique en la pression artérielle moyenne au même titre que l'onde dicrote et de manière complémentaire. En effet, le PI évolue en général dans le sens opposé de la pression artérielle moyenne.

On utilise une mesure que l'on a dénommée mPI (pour PI modifié) et qui est calculée comme suit : mPI=10 x In(1/PI+1). Le mPI ainsi obtenu varie dans le même sens que la pression artérielle moyenne est le comportement se retrouve linéarisé par rapport au comportement exponentiel du PI.

On effectue une calibration *Calib* avec la pression artérielle moyenne mesurée et le mPl au temps 0 et on calcule la pression artérielle moyenne au temps t en utilisant PAMest = *Calib* x mPl(t).

### Utilisation de plusieurs paramètres

On peut utiliser plusieurs paramètres (hauteur de l'onde dicrote, mPl, durées indiquées ci-dessus).

On peut
- calculer régulièrement un Calib pour chaque paramètre
- calculer la PAMest pour chacun des paramètres
- définir la PAMest par moyenne statistique (filtre de Kalman) en pondérant ces valeurs PAMest et utilisant la valeur calculée au temps précédent

### Exemple 3. Exemplification en conditions réelles

Ces résultats ont été obtenus sur la base de la hauteur de l'onde dicrote, des résultats similaires peuvent être obtenus avec les autres paramètres.

On a réalisé une étude au bloc opératoire de neurochirurgie ou au cours d'une procédure de neuroradiologie interventionnelle. Les patients ont bénéficié de la prise en charge habituelle de base pour ce type d'intervention incluant :

### Monitorage

- Un monitorage hémodynamique non invasif de la pression artérielle par oscillometrie, ainsi qu'une surveillance continue de l'ECG
- Un monitorage continue de la saturation pulsée en oxygène (SpO2) par photopléthysmographie, ainsi qu'un monitorage du CO2 expiré.
- Un monitorage de la profondeur de l'anesthésie par l'index bispectral (BIS)
- Induction et entretien de l'anesthésie générale selon une méthode intraveineuse à objectif de concentration (AIVOC) incluant propofol et remifentanil, et curarisation avant intubation oro-trachéale par bésilate d'atracurium.
- L'ensemble des moniteurs était relié à un moniteur Philips.
- l'hypotension artérielle a été définie par une baisse de pression artérielle moyenne (PAM) d'au moins 20 % par rapport à la PAM basale.
- Lors d'une hypotension artérielle constatée l'anesthésiste en charge du patient était libre d'alléger l'anesthésie, d'administrer un remplissage vasculaire ou un vasoconstricteur (Ephedrine 9mg,Phényéphrine 50mcg ou Noradrénaline 10 mcg).

### Protocole expérimental

### Phase 1 : Préoxygénation (baseline) - Induction anesthésique.

- Pré-oxygénation pendant 2min : C'est durant cette phase et avant toute injection que sont enregistrées les valeurs de base de tous les paramètres (moyenne de 2 valeurs, baseline )
- Rémifentanil à 5 ng/ml de concentration cible pendant 1 min.
- Propofol 5 µg/ml de concentration cible.
- Après que le BIS soit descendu en dessous de 50 et vérification de l'abscence de reflexe ciliaire et que le patient soit ventilable manuellement : curarisation par 0.5 mg/kg de tracrium.
- Attente de 3 min avec ventilation manuelle.

### Phase 2 : Laryngoscopie-Intubation-Ventilation manuelle.

- Laryngoscopien directe.
- Intubation orotrachéale.
- Ventilation Manuelle et fixation de la sonde.

### Phase 3 : Ventilation Mécanique-Entretien anesthésie.

- Branchement du patient au respirateur et mise en route de la ventilation mécanique.
- Baisse des cibles du Remifentanil et Propofol à 3.5 ng/ml et 4 µg/ml respectivement.
- Poursuite du recueil pendant 3 min.

### Phase 4 : Correction Hypotension éventuelle par vasoconstricteur.

- Episode hypotensif traité par administration de vasoconstricteur.
- Poursuite du recueil une minute après effet du vasoconstricteur.
- Fin du recueil.

Durant les phases 1,2 et début de la 3 la pression brassard était prise toutes les minutes pour une durée moyenne estimée de 15 min. L'anesthésiste en charge du patient était libre de dévier du protocole initial à tout moment s'il jugeait que la situation clinique le nécessitait.

Le recueil des données a été réalisé à l'aide du logiciel de saisie de données Extrend (Ixellence) recueillant les signaux à une fréquence de 125Hz et toutes les valeurs numériques.

Un point de recueil de tous les paramètres suivants était réalisé toutes les minutes :
- Hauteur de l'onde dicrote : le calcul de la hauteur de l'onde dicrote sur le signal de plétysmographie.
- IP : l'indice de perfusion était recueilli battement par battement.

### Résultats:

61 patients ont été inclus dans l'étude (55 ans d'âge médian, 32,7% d'hommes / 67,3% de femmes).

54 patients sur 61 ont eu au moins un épisode d'hypotension. L'incidence de l'hypotension, définie par une baisse de la PAM>20%, dans la population était de 88,5%). Le temps passé avec une PAM<20% est en moyenne de 5,2 min au cours de l'induction soit 44% du temps.

### Évolution des valeurs au cours de la totalité de l'induction.

La durée moyenne de l'ensemble de la phase d'induction était de 12 ± 4 min.

### Evolution PAM et hauteur de l'onde dicrote

Les variations de PAM et les variations de la hauteur de l'onde dicrote étaient fortement corrélées de façon linéaire sur toute la durée de l'induction (voir figure 7, notamment la stabilité de la valeur Calib (figure 7.A)).

### Analyse sur la base de mesure de pression artérielle moyenne en continu par cathétérisme artériel invasif.

Le cathéter artériel est un dispositif permettant un accès artériel afin de mesurer la pression artérielle, de manière invasive et continue, et de réaliser des prélèvements de sang artériel.

La pression artérielle sanglante ou invasive, est une technique invasive de monitorage de la pression artérielle intra-vasculaire par un cathéter artériel.

On a effectué une mesure en continu de la pression artérielle par cathéter artériel et par le procédé selon l'invention (calcul via la hauteur de l'onde dicrote mesurée par pléthysmographie).

On a observé une parfaite corrélation des variations de la PAM mesurée par cathéter artériel et par la méthode et cela après utilisation de médicaments vasopresseurs. La corrélation était de r=0.88 avec une concordance de 96% entre les variations obtenues par la technique et la mesure réelle de PAM (Figure 7.A).

Dans la figure 7.A, on peut voir notamment à la fin une variation significative de pression qui n'est pas détectée par le brassard (du fait du temps entre deux prises de pression), mais l'est par le signal PlethoMAP, et qui souligne donc le caractère informatif de la méthode utilisant l'onde dicrote.

Dans la figure 7.B, on voit bien que seule la mesure de la hauteur de l'onde dicrote permet d'obtenir une valeur de la PAM, et que l'évolution des deux autres paramètres (valeur systolique ou valeur diastolique) n'est pas suffisamment informative. On peut aussi voir (T = 1,15h) que la baisse de hauteur de l'onde dicrote est indicatrice de la baisse réelle de PAM (figure 7.A), alors que les pressions systoliques et diastoliques ne varient pas. La combinaison de ces deux pressions n'aurait donc pas permis de détecter la baisse de pression artérielle moyenne, et de permettre au médecin anesthésiste de prendre toute mesure corrective.

### Exemple 4. Autres paramètres

Une étude a été menée sur des patients, en accord avec les règles applicables. Les patients étaient âgés de plus de 18 ans, et qui subissaient des interventions neuroradiologiques électives, après consentement éclairé. Les critères d'exclusion de l'étude étaient l'arythmie cardiaque (c.-à-d. la fibrillation auriculaire) et la grossesse.

### Protocole d'anesthésie

Avant l'induction de l'anesthésie, la surveillance standard a été initiée par électrocardiogramme, une mesure non invasive de l'ABP brachial (PHILIPS FRANCE, Suresnes, France) réglée pour se gonfler chaque minute et une oxymétrie de pouls numérique (PHILIPS FRANCE, Suresnes, France) placée sur le deuxième doigt du côté controlatéral au brassard de l'ABP. L'index bispectral (BIS^{™}quatro sensor, Medtronic France, Boulogne-Billancourt, France) et le suivi du blocage neuromusculaire (TOF Watch^{®}, ALSEVIA PHARMA, Paris, France) ont également été utilisés pour surveiller l'anesthésie. Tous les paramètres de surveillance étaient disponibles sur un moniteur PHILIPS Intellivue MP 60 (PHILIPS FRANCE, Suresnes, France). L'induction de l'anesthésie a été réalisée au moyen de rémifentanil et de propofol avec une dose initiale de 5ng mL⁻¹ et 5µg mL⁻¹ respectivement, et ajustée pour atteindre un BIS entre 40 et 60. Après que le BIS a diminué sous 60 et la perte de conscience, le blocage neuromusculaire a été effectué par injection intraveineuse de 0,5mg kg⁻¹ d'Atracurium. Les patients ont ensuite été ventilés mécaniquement par intubation trachéale par laryngoscopie directe (volume en fin d'expiration = 6mL kg⁻¹ de poids corporel idéal, pression expiratoire finale positive = 5cmH₂O, fréquence respiratoire et fraction oxygène pour atteindre un CO₂ en fin d'expiration = 4,7 kPa et une saturation en O₂ >95%).

La pression artérielle a été mesurée toutes les minutes pendant l'induction et toutes les 5 minutes après l'intubation trachéale et la stabilisation. L'anesthésiste responsable du patient peut à tout moment modifier la fréquence des mesures et traiter les épisodes d'IOH avec charge liquidienne et/ou vasopresseurs (phényléphrine et/ou norépinéphrine). Après l'induction, certains patients pouvaient également bénéficier d'une surveillance invasive et continue de la tension artérielle.

### Collecte des données

Tous les paramètres et courbes de surveillance affichés sur l'écran ont été enregistrés sur un ordinateur. Les paramètres hémodynamiques (fréquence cardiaque, pression artérielle systolique [SAP], pression artérielle moyenne [MAP] et pression artérielle diastolique [DAP]) et les paramètres PPG (Dicpleth, PI et SpO₂) ont ensuite été échantillonnés rétrospectivement chaque minute pendant l'induction. La période d'induction a été définie arbitrairement de la pré-oxygénation à 3 min après le raccordement à la ventilation mécanique. Les valeurs de référence ont été obtenues en faisant la moyenne des deux mesures (espacées d'une minute) avant l'injection des anesthésiques pendant la période de préoxygénation. Les valeurs de "prépresseur" ont été définie comme étant les mesures antérieures au bolus vasopresseur pendant les épisodes d'IOH. "Les valeurs de "crête-pression" ont été définies comme les effets maximaux du bolus vasopresseur, lorsque la PMA la plus élevée a été atteinte. En accord avec la plupart des études, l'IOH a été défini comme une baisse de plus de 20 % du PAM de référence.

### Dicpleth et mesure PI

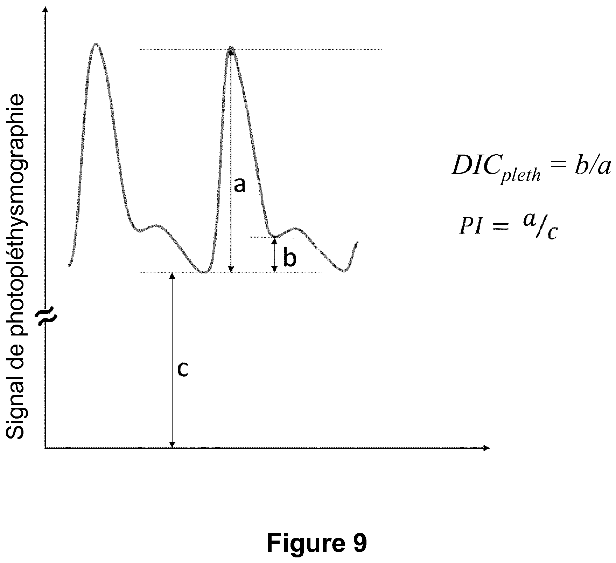

Dicpleth a été obtenu a posteriori à partir des formes d'onde PPG enregistrées par un opérateur aveugle aux valeurs ABP. Dicpleth a été défini comme le rapport de la hauteur de l'encoche dicrotique (du point nadir du complexe à l'encoche) à la hauteur du pic systolique (du même point nadir du complexe à l'image), mesuré en fin de temps expiratoire chez des patients sous ventilation mécanique (moyenne de 3 complexes consécutifs) (Figure 9). L'IP (indice de perfusion) a été fourni par le fabricant et est calculé comme le rapport de la composante pulsatile du signal PPG et de la composante continue (figure 9).

ΔMAP, ΔDicpleth et ΔPI ont été calculés pendant la période d'induction comme étant leurs variations relatives (en pourcentage) par rapport à leurs valeurs de référence. Pendant les bolus vasopresseurs, les variations des paramètres respectifs ont été calculées entre les mesures "prépresseur" et "pic-presseur".

### Mesure dicradiale

Chez les patients présentant une surveillance invasive pendant le maintien de l'anesthésie, Dicradial a également été mesuré à partir du signal de pression artérielle avec la même méthodologie que Dicpleth. Les 3 derniers battements cardiaques de la fin de la période expiratoire ont été utilisés pour calculer Dicradial, de la hauteur de l'encoche dicrotique à la hauteur du pic systolique. Chez ces patients, Dicpleth, Dicradial et leurs variations relatives (ΔDicpleth et ΔDicradial) pendant l'administration des vasoconstricteurs ont également été analysés.

### Analyse statistique

Les valeurs ont été exprimées sous forme d'écart médian et interquartile [25e et 75e percentiles]. Les modifications des paramètres ont été analysées à l'aide du test de classement de Wilcoxon. Les pourcentages de concordance entre le delta ΔMAP, et ΔDicpleth et ΔPI ont été calculés pendant la période d'induction. Les aires sous la courbe (ASC) de la courbe des caractéristiques de fonctionnement du récepteur (ROC) (avec un intervalle de confiance de 95 %) de ΔDicpleth et ΔPI pour détecter les épisodes d'IOH ont été estimées et éventuellement comparées en utilisant le test DeLong. La méthode Youden a été utilisée pour déterminer les valeurs seuils optimales de ΔDicpleth et de ΔPI pour détecter les épisodes d'IOH. La courbe ROC de combinaison de ΔDicpleth et ΔPI a été construite en utilisant le modèle logistique. Les tests de corrélation entre les deux ont été effectués à l'aide du test Spearman. P<0,05 a été considéré comme statistiquement significatif. L'objectif principal de l'étude était d'estimer l'ASC de la courbe ROC de ΔDicpleth et ΔPI pour suivre l'IOH pendant l'induction. La taille de l'échantillon a été déterminée avec une ASC attendue à 0,85, une incidence prévue d'hypotension de 80 % et une largeur de l'intervalle de confiance de 1. Avec une puissance de 80 %, le nombre de patients à inclure était alors de 62,16 L'objectif secondaire était d'évaluer l'ASC de la courbe ROC (AUROC) pour la combinaison ΔDicpleth et ΔPI. L'analyse statistique a été effectuée à l'aide de Prism 6.00^{©} (Graphpad Software, Inc, La Jolla, CA, USA) et R 3.3.0 (R foundation for Statistical Computing, Vienne, Autriche). Les patients qui présentaient un Dicpleth non mesurable au départ avant l'induction de l'anesthésie ont été exclus de l'analyse.

### Résultats

De novembre 2014 à mai 2015, 65 patients ont été inclus dans l'étude. Avant l'induction de l'anesthésie, Dicpleth n'était pas mesurable chez 4 patients (6,2 %) en raison de l'absence d'entaille dicrotique détectable sur le signal PPG (onde de classe IV selon Dawber et al). La plupart des patients étaient atteints d'AAS II, avec un âge moyen de 54[39 ; 64] ans. L'hypertension artérielle, le tabagisme et la dyslipidémie étaient les comorbidités les plus fréquentes. La raison des procédures de neuroradiologie était principalement due à un anévrisme ou à une malformation artério-veineuse avec embolisation programmée.

### Evolution des MAP, Dicpleth et PI pendant la période d'induction

La durée médiane de l'induction de l'anesthésie était de 11 [10 ; 13,5] minutes. Au total, 720 "points de données hémodynamiques" ont été enregistrés : 61 au départ et 659 après injection anesthésique, soit 659 variations par rapport au départ.

La valeur de référence du MAP était de 86 [79 ; 93] mmHg, ce qui donne une limite individuelle de IOH à 69 [62 ; 74] mmHg. La PMA a diminué à 54 [48 ; 60] mmHg avant la laryngoscopie et est passée à 72 [64 ; 82] mmHg après l'intubation trachéale. La PAM moyenne sur la période d'induction globale était de 70 [64 ; 71] mmHg. Cinquante-quatre patients (88 %) ont souffert d'au moins un épisode d'IOH lors de l'induction de l'anesthésie, soit 323 mesures (49 % des points hémodynamiques). Vingt-huit patients (46 %) ont reçu un bolus de vasopresseurs pendant l'induction (2 phényléphrine et 26 noradrénaline).

Le Dicpleth était à 0,54 [0,45 ; 0,65] au départ, est tombé à 0,36 [0,19 ; 0,45] (p<0,001) et a augmenté à 0,46 [0,41 ; 0,56] après intubation trachéale. Les valeurs de base de l'IP étaient de 1,7 [0,9 ; 3] et ont augmenté à 4,4 [2,8 ; 6,6] (p<0,001) avant la laryngoscopie, puis diminué à 3,6 [2,1 ; 5,4] après intubation trachéale. Une représentation visuelle de ΔMAP, ΔDicpleth et ΔPI pendant la période d'induction est décrite à la figure 10. Il a été trouvé 89% de concordance entre ΔDicpleth et ΔMAP et 90% entre ΔPI et ΔMAP (ESM1).

### Performances diagnostiques de ΔDicpleth et ΔPI pour la détection de l'hypotension artérielle

Les valeurs des performances de diagnostic de ΔDicpleth et ΔPI sont résumées dans le tableau 1.

**Tableau 1**

| | **ΔDicₚₗₑₜₕ** | **ΔPI** | **ΔDicₚₗₑₜₕ+ ΔPI** |
|---|---|---|---|
| **ASC ROC** | 0.83 | 0.86 | 0.91 |
| | 95%CI 0.80-0.86) | (95%CI 0.80-0.86) | (95%CI 0.88-0.95) |
| P-value | <0.001 | <0.001 | <0.001 |
| **Valeur seuil** | -19% | 51% | NA |
| **Sensibilité (%)** | 79 | 82 | 84 |
| **Spécificité (%)** | 84 | 74 | 84 |
| **VPP (%)** | 79 | 71 | 79 |
| **VPN (%)** | 84 | 85 | 89 |

| | | | |
|---|---|---|---|
| ΔDicpleth : variation relative de Dicpleth par rapport à la ligne de base ; ΔPI : variation relative de l'indice de perfusion par rapport à la ligne de base ; ASC ROC : aire sous la « Receiving Operative Curve »; PPV : valeur prédictive positive ; NPV : valeur prédictive négative | | | |

Les meilleures valeurs seuils de ΔDicpleth et ΔPI pour la détection de l'OHI étaient de -19% et 51%, respectivement. Les ASC de ΔDicpleth et ΔPI n'étaient pas significativement différentes (p=0.22). La combinaison de ΔDicpleth et de ΔPI pour détecter les épisodes d'homéostasie intra-utérine a amélioré la performance de détection avec une ASC de la courbe ROC (0,91, (95 %CI 0,88-0,95, p<0,001) statistiquement meilleure que ΔDicpleth et ΔPI séparément (p=0,026 et p<0,001, respectivement).

### Évolution de la MAP, de la Dicpleth et de l'IP pendant l'administration vasoconstrictrice

Vingt-huit patients (46 %) ont reçu un bolus de vasopresseurs pendant l'induction (2 phényléphrine et 26 noradrénaline). Après les vasopresseurs, la MAP est passée de 59 [50 ; 67] mmHg à 76 [68 ; 79] mmHg (variation relative : 30 % [14 ; 45], p<0,001). Le nombre de personnes atteintes de diabète a augmenté de 0,34 [0,25 ; 0,39] à 0,48 [0,35 ; 0,55] (variation relative : 44 % [17 ; 63], p<0,001) et l'IP a diminué de 4,0 [3,3 ; 5,4] à 3,2 [1,8 ; 5,4] (variation relative : -28 % [-44;-13], p<0,001). ΔDicpleth et ΔPI sous l'effet des vasopresseurs étaient fortement liés à ΔMAP (r=+0,73, 95%CI 0,48-0,87, p<0,001 et r = -0,62 95%CI -0,81 à -0,32, p<0,001 ; respectivement).

### Évolution Dicpleth et Dicradial pendant l'administration vasoconstrictrice

Pendant le maintien de l'anesthésie, 48 bolus de norépinéphrine ont été administrés chez 10 patients (5 [4 ; 6] bolus par patient) sous surveillance invasive de la tension artérielle. Le Dicpleth n'était pas mesurable sur 2 points hémodynamiques qui ont été exclus de l'analyse. La MAP est passé de 70 [63 ; 77] mmHg à 88 [77 ; 98] mmHg (variation relative 26% [19 ; 34], p<0.001). Le Dicpleth a augmenté de 0,28 [0,17 ; 0,36] à 0,39 [0,25 ; 0,46] et le Dicradial de 0,32 [0,21 ; 0,39] à 0,40 [0,31 ; 0,49] (variations relatives respectives 34% [20 ; 71], p<0,001 et 27% [14 ; 46], p<0,001). Dicpleth et Dicradial et leurs variations relatives étaient fortement corrélées pendant l'administration vasoconstrictrice (r=0,87 95%CI 0,83-0,90 et r=0,92 95%CI 0,85-0,95).

Ces résultats montrent que Dicpleth peut être utilisé comme paramètre de substitution pour la surveillance non invasive et continue de la MAP pendant l'induction de l'anesthésie. Ces résultats montrent une forte corrélation entre ΔDicpleth et ΔMAP sous l'action des vasoconstricteurs. Une baisse de 19% de Dicpleth avait de bonnes performances pour détecter l'IOH avec une sensibilité de 79% et une spécificité de 84%. PI représente le rapport entre les composantes pulsatile et continue de l'absorption lumineuse. Les résultats montrent une corrélation négative entre ΔPI et ΔMAP sous vasopresseurs. ΔPI était également précis pour détecter l'IOH, mais probablement un peu moins que ΔDicpleth pour informer sur l'intensité de l'IOH.

## Revendications

1. Méthode mise en œuvre par ordinateur pour évaluer en continu la pression artérielle moyenne d'un patient, sur la base de valeurs reçues en continu d'un paramètre *Vp* ayant été mesuré par pléthysmographie, comprenant les étapes :
I. Calculer une valeur de calibration *Calib* à partir
a. De la valeur de la pression artérielle moyenne mesurée à un temps t0
b. De la valeur *Vp0* liée audit paramètre, mesurée chez le patient au temps t0, la valeur V*p0* du paramètre étant la hauteur de l'onde dicrote au temps t0
II. Calculer la valeur estimée PAMest de la pression artérielle du patient à un temps t postérieur à t0 par la formule PAMest = *Calib x* V*pt,* dans laquelle *Vpt* est la valeur de la mesure du paramètre obtenue au temps t, la valeur *Vpt* du paramètre étant la hauteur de l'onde dicrote au temps t.

2. Méthode selon la revendication 1, **caractérisée en ce que** la valeur *Vpt* du paramètre a été obtenue par photopléthysmographie prise sur le doigt ou le lobe de l'oreille.

3. Méthode selon la revendication 1 ou 2, **caractérisée en ce que** l'on calcule également la valeur de l'indice de perfusion, de l'inverse de l'indice de perfusion ou du logarithme de l'inverse de l'indice de perfusion + 1.

4. Méthode selon l'une des revendications 1 à 3, **caractérisée en ce que** la valeur *Vpt* utilisée est une valeur moyennée à partir de plusieurs valeurs mesurées sur une durée prédéterminée.

5. Méthode selon l'une des revendications 1 à 4, **caractérisée en ce que** l'on recalcule la valeur Calib de temps à autre, en particulier à intervalles réguliers par mesure d'une nouvelle valeur de la pression artérielle et mesure d'une nouvelle valeur du paramètre Vp, et que l'on utilise cette nouvelle valeur Calib ultérieurement,

6. Méthode selon l'une des revendications 1 à 5, **caractérisée en ce qu'**elle est effectuée pendant toute la durée d'anesthésie générale d'un patient.

7. Méthode selon l'une des revendications 1 à 6, **caractérisée en ce que** la hauteur de l'onde dicrote est calculée par :
(a) Détermination du pied de l'onde de pouls en
- calculant la seconde dérivée du signal de pléthysmographie, le pied de l'onde de pouls correspondant au maximum de cette seconde dérivée, au niveau du front ascendant de l'onde de pouls.
(b) Détermination du pic de l'onde de pouls, après le pied de l'onde de pouls en
- Découpant le signal en plusieurs fenêtres de temps (en particulier des fenêtres entre 20 ms et 100 ms, en particulier de 50 ms), et
- regardant la valeur maximale dans chaque fenêtre de temps, la valeur maximale locale correspondant à la valeur V maximale trouvée dans une fenêtre de temps, la valeur maximale de la fenêtre de temps suivant étant inférieur à cette valeur V correspondant au pci de l'onde de pouls,
(c) Détermination de la hauteur de l'onde dicrote par
- obtention de la seconde dérivée du signal après le pic de l'onde de pouls
- découpage du signal sur des fenêtres de temps prédéterminées (entre 50 ms et 300 ms, en particulier de 150 ms) après ce pic de l'onde de pouls
- recherche du maximum local de la seconde dérivée du signal dans chaque fenêtre de temps, afin de déterminer une zone d'intérêt, qui est la fenêtre de temps dans laquelle on a ce maximum local de la seconde dérivée du signal
- Recherche du minimum en valeur absolue de la première dérivée du signal dans cette zone d'intérêt, le point correspondant à l'onde dicrote étant à ce point pour lequel on atteint le minimum en valeur absolue de la première dérivée,
- Mesure de la hauteur de l'onde dicrote en ce point.

8. Dispositif pour la mise en oeuvre d'une méthode selon l'une des revendications 1 à 7, comprenant :
- des moyens de réception de données de mesure de la pression artérielle moyenne
- des moyens de réception de données de mesure de la hauteur de l'onde dicrote, mesurée en continu
- des moyens de calcul permettant de calculer un coefficient Calib à chaque mesure réelle de pression artérielle moyenne
- des moyens de calcul permettant de calculer une pression artérielle moyenne à chaque instant t en fonction de la valeur des paramètres à cet instant t, par application de la méthode selon l'une des revendications 1 à 7
- des moyens de présentation de la pression artérielle moyenne calculée à chaque instant t, incluant éventuellement des moyens d'alerte dans le cas où la pression artérielle moyenne calculée est inférieure à une valeur prédéterminée
- éventuellement des moyens permettant l'actionnement automatique du dispositif mesurant la pression artérielle moyenne, dans le cas où la pression artérielle moyenne calculée est inférieure à une valeur prédéterminée
- éventuellement des moyens permettant l'administration automatique d'une dose de vasopresseur, dans le cas où la pression artérielle moyenne calculée ou estimée est inférieure à une valeur prédéterminée.

9. Produit/programme d'ordinateur pour comprenant des instructions de code de programme enregistrées sur un support lisible par un ordinateur, pour mettre en oeuvre les étapes du procédé selon l'une des revendications 1 à 7, lorsque ledit programme est exécuté sur un ordinateur.

10. Support d'enregistrement lisible par un ordinateur sur lequel est enregistré un programme d'ordinateur comprenant des instructions de code de programme pour l'exécution des étapes du procédé selon l'une des revendications 1 à 7.

## Patentansprüche

1. Ein computergestütztes Verfahren zur kontinuierlichen Bewertung des mittleren arteriellen Blutdrucks eines Patienten auf der Grundlage kontinuierlich empfangener Werte eines durch Plethysmographie gemessenen Parameters *Vp,* das die folgenden Schritte umfasst:
I. Berechnen eines Kalibrierungswerts *Calib* aus
a. dem Wert des mittleren arteriellen Drucks, gemessen zu einem Zeitpunkt t0
b. des mit diesem Parameter verbundenen Wertes *Vp0,* gemessen beim Patienten zum Zeitpunkt t0, wobei der Wert *Vp0* des Parameters die Höhe der dikrotischen Welle zum Zeitpunkt t0 ist
II. Berechnen des geschätzten Wertes MAPest des arteriellen Drucks des Patienten zu einem Zeitpunkt t nach t0 nach der Formel MAPest *= Calib x Vpt,* wobei *Vpt* der Wert der zum Zeitpunkt t erhaltenen Parametermessung ist, wobei der Wert *Vpt* des Parameters die Höhe der dikrotischen Welle ist.

2. Verfahren nach Anspruch 1, **dadurch gekennzeichnet, dass** der Wert *Vpt* des Parameters durch Photoplethysmographie am Finger oder Ohrläppchen ermittelt wurde.

3. Verfahren nach Anspruch 1 oder 2, **dadurch gekennzeichnet, dass** auch der Wert des Perfusionsindex, der Kehrwert des Perfusionsindex oder der Logarithmus des Kehrwerts des Perfusionsindex + 1 berechnet wird.

4. Verfahren nach einem der Ansprüche 1 bis 3, **dadurch gekennzeichnet, dass** der verwendete Wert *Vpt* ein Mittelwert aus einer Vielzahl von Messwerten über einen vorbestimmten Zeitraum ist.

5. Das Verfahren nach einem der Ansprüche 1 bis 4, **dadurch gekennzeichnet, dass** der Wert Calib von Zeit zu Zeit, insbesondere in regelmäßigen Abständen, durch Messen eines neuen arteriellen Druckwertes und Messen eines neuen Wertes des Parameters Vp neu berechnet wird und dass dieser neue Wert Calib anschließend verwendet wird.

6. Das Ex-vivo-Verfahren nach einem der Ansprüche 1 bis 5, **dadurch gekennzeichnet, dass** es während der gesamten Dauer der Vollnarkose eines Patienten durchgeführt wird.

7. *Ex-vivo-Verfahren* gemäß einem der Ansprüche 1 bis 6, **dadurch gekennzeichnet, dass** die Höhe der dikrotischen Welle berechnet wird durch:
(a) Bestimmung des Pulswellenfußes durch
- Berechnung der zweiten Ableitung des Plethysmographiesignals, wobei der Pulswellenfuß dem Maximum dieser zweiten Ableitung an der steigenden Flanke der Pulswelle entspricht.
(b) Bestimmung des Peaks der Pulswelle nach dem Fuß der Pulswelle durch
- Aufteilung des Signals in mehrere Zeitfenster (insbesondere Fenster zwischen 20 ms und 100 ms, insbesondere 50 ms) und
- Betrachtung des Maximalwerts in jedem Zeitfenster, wobei der lokale Maximalwert dem in einem Zeitfenster gefundenen Maximalwert V entspricht, wobei der Maximalwert des folgenden Zeitfensters kleiner ist als dieser Wert V, der dem Scheitelpunkt der Pulswelle entspricht,
(c) Bestimmung der Höhe der dikrotischen Welle durch
- Ermitteln der zweiten Ableitung des Signals nach dem Scheitelpunkt der Pulswelle
- Aufteilen des Signals in vorbestimmte Zeitfenster (zwischen 50 ms und 300 ms, insbesondere 150 ms) nach diesem Peak der Pulswelle
- Suche nach dem lokalen Maximum der zweiten Ableitung des Signals in jedem Zeitfenster, um einen interessierenden Bereich zu bestimmen, der das Zeitfenster ist, in dem dieses lokale Maximum der zweiten Ableitung des Signals gefunden wird
- Suchen des absoluten Minimums der ersten Ableitung des Signals in diesem interessierenden Bereich, wobei der Punkt, der der dikrotischen Welle entspricht, der Punkt ist, an dem das absolute Minimum der ersten Ableitung erreicht wird
- Messen der Höhe der dikrotischen Welle an diesem Punkt.

8. Vorrichtung zur Durchführung eines Verfahrens gemäß einem der Ansprüche 1 bis 7, umfassend:
- Mittel zum Empfangen von Messdaten des mittleren arteriellen Drucks
- Mittel zum Empfangen von Messdaten der kontinuierlich gemessenen Höhe der dicrotischen Welle
- Berechnungsmittel zum Berechnen eines Koeffizienten Calib für jede tatsächliche Messung des mittleren arteriellen Drucks
- Berechnungsmittel zum Berechnen eines mittleren arteriellen Drucks zu jedem Zeitpunkt t als Funktion des Wertes der Parameter zu diesem Zeitpunkt t unter Anwendung des Verfahrens gemäß einem der Ansprüche 1 bis 7
- Mittel zum Anzeigen des berechneten mittleren arteriellen Drucks zu jedem Zeitpunkt t, optional einschließlich Mitteln zum Auslösen eines Alarms, falls der berechnete mittlere arterielle Druck unter einem vorbestimmten Wert liegt
- optional Mittel zur automatischen Betätigung der Vorrichtung zur Messung des mittleren arteriellen Drucks, falls der berechnete mittlere arterielle Druck unter einem vorgegebenen Wert liegt
- optional Mittel zur automatischen Verabreichung einer Dosis eines Vasopressors, falls der berechnete oder geschätzte mittlere arterielle Druck unter einem vorbestimmten Wert liegt.

9. Ein Computerprodukt/Programm, das auf einem computerlesbaren Medium aufgezeichnete Programmcodeanweisungen umfasst, um die Schritte des in einem der Ansprüche 1 bis 7 beanspruchten Verfahrens auszuführen, wenn das Programm auf einem Computer ausgeführt wird.

10. Ein computerlesbares Aufzeichnungsmedium, auf dem ein Computerprogramm aufgezeichnet ist, das Programmcodeanweisungen zum Ausführen der Schritte des in einem der Ansprüche 1 bis 7 beanspruchten Verfahrens umfasst.

## Claims

1. A computer implemented method for continuously evaluating the mean arterial pressure of a patient, based on continuously received values of a parameter *Vp* measured by plethysmography, comprising the steps:
I. Calculate a calibration value *Calib* from
a. The value of the mean arterial pressure measured at a time t0
b. The value *Vp0* linked to said parameter, measured in the patient at the time t0, the value *Vp0* of the parameter being the height of the dicrotic wave at time t0
II. Calculate the estimated value MAPest of the patient's arterial pressure at a time t after t0 by the formula MAPest = *Calib x Vpt,* wherein *Vpt* is the value of the parameter measurement obtained at the time t, the value *Vpt* of the parameter being the dicrotic wave height.

2. The method as claimed in claim 1, **characterized in that** the value *Vpt* of the parameter has been obtained by photoplethysmography taken from the finger or earlobe.

3. The method as claimed in claim 1 or 2, **characterized in that** the value of the perfusion index, the inverse of the perfusion index or the logarithm of the inverse of the perfusion index + 1 is also calculated.

4. The method as claimed in one of claims 1 to 3, **characterized in that** the value *Vpt* used is a value averaged from a plurality of measured values over a predetermined period of time.

5. The method as claimed in one of claims 1 to 4, **characterized in that** the value Calib is recalculated from time to time, in particular at regular intervals by measuring a new arterial pressure value and measuring a new value of the parameter Vp, and that this new value Calib is used subsequently.

6. The *ex vivo* method as claimed in one of claims 1 to 5, **characterized in that** it is carried out during the entire period of general anesthesia of a patient.

7. Method *ex vivo* according to one of claims 1 to 6, **characterized in that** the height of the dicrotic wave is calculated by:
(a) Determination of the pulse wave foot by
- calculating the second derivative of the plethysmography signal, the pulse wave foot corresponding to the maximum of this second derivative, at the rising edge of the pulse wave.
(b) Determination of the peak of the pulse wave, after the foot of the pulse wave, by
- splitting the signal into several time windows (in particular windows between 20 ms and 100 ms, in particular 50 ms), and
- looking at the maximum value in each time window, the local maximum value corresponding to the maximum value V found in a time window, the maximum value of the following time window being less than this value V corresponding to the peak of the pulse wave,
(c) Determining the height of the dicrotic wave by
- obtaining the second derivative of the signal after the peak of the pulse wave
- splitting the signal into predetermined time windows (between 50 ms and 300 ms, in particular 150 ms) after this peak of the pulse wave
- searching for the local maximum of the second derivative of the signal in each time window, in order to determine an area of interest, which is the time window in which this local maximum of the second derivative of the signal is found
- searching for the absolute minimum of the first derivative of the signal in this area of interest, the point corresponding to the dicrotic wave being the point at which the absolute minimum of the first derivative is reached
- Measuring the height of the dicrotic wave at this point.

8. Device for implementing a method according to any one of claims 1 to 7, comprising:
- means for receiving mean arterial pressure measurement data
- means for receiving measurement data of the dicrotic wave height, measured continuously
- calculation means to calculate a coefficient Calib for each actual measurement of mean arterial pressure
- calculation means to calculate a mean arterial pressure at each time t as a function of the value of the parameters at that time t, by applying the method according to any one of claims 1 to 7
- means for presenting the calculated mean arterial pressure at each time t, optionally including means for alerting in the event that the calculated mean arterial pressure is below a predetermined value
- optionally means for the automatic actuation of the device for measuring the mean arterial pressure, in the event that the calculated mean arterial pressure is below a predetermined value
- optionally means for the automatic administration of a dose of vasopressor, in the event that the calculated or estimated mean arterial pressure is below a predetermined value.

9. A computer product/program for comprising program code instructions recorded on a computer-readable medium, for carrying out the steps of the process as claimed in one of claims 1 to 7, when said program is executed on a computer.

10. A computer-readable recording medium on which is recorded a computer program comprising program code instructions for performing the steps of the process as claimed in one of claims 1 to 7.
